# EUROPEAN PATENT SPECIFICATION

(11) **EP 4 251 122 B1**
(45) Date of publication and mention of the grant of the patent: **12.03.2025**
(21) Application number: 22701777.9
(22) Date of filing: 06.01.2022
(51) Int. Cl.: A61K 8/33, A61K 8/42, A61K 8/34, A61Q 19/00, A61Q 11/00, A61Q 5/00, A23L 2/56, A23L 27/20

(54) **COMPOSITIONS THAT ENHANCE THE COOLING EFFECT**
ZUSAMMENSETZUNGEN ZUR VERSTÄRKUNG DER KÜHLWIRKUNG
COMPOSITIONS AMÉLIORANT L'EFFET DE RAFRAÎCHISSEMENT

(30) Priority: 13.01.2021 US 202163136847 P; 18.02.2021 US 202163150654 P
(43) Date of publication of application: 04.10.2023
(73) Proprietor: Firmenich Incorporated, Plainsboro, NJ 08536 (US)
(72) Inventor: PRIEST, Chad, San Diego, California 92121 (US); OUYANG, Qing-Bo, Plainsboro, New Jersey 08536 (US); ASHOKAN, Bharani, Plainsboro, New Jersey 08536 (US); HAINES, Jan, Thomas, Singapore 638377 (SG); MERCERET, Patrice, 1242 Satigny (CH); MITHAIWALLA, Parizad, Singapore 638377 (SG); VIGOUROUX, Florence, 1242 Satigny (CH); LEDERREY, Pauline, 1242 Satigny (CH)
(74) Representative: Strych, Sebastian
(86) International application number: PCT/US2022/011513
(87) International publication number: WO 2022/155054

(56) References cited:
- US-A1- 2015 297 753
- US-A1- 2016 376 263

## Description

### TECHNICAL FIELD

The present disclosure generally relates to coolness-modifying compositions that enhance the cooling effect of cooling compounds, such as menthol, cubebol, and the like. In some aspects, the disclosure provides uses of such coolness-modifying compositions to cooling effect of cooling compounds. In some other aspects, the disclosure provides compositions (such as comestible compositions or oral care compositions), which comprise a cooling compound (for example, menthol, cubebol, and the like) and a coolness-modifying composition of the present disclosure. In some embodiments, such compositions are in the form of a food product, a beverage product, or an oral care product, such as a toothpaste, a mouthwash, a tooth-whitening composition, a chewing gum, a dentifrice, and the like.

### DESCRIPTION OF RELATED ART

Our sensory systems are able to detect subtle changes in ambient temperature, due to the coordinated efforts of certain thermosensory neurons. Temperature-sensitive proteins of the TRP channel family are typically found at the site at which thermal stimuli are converted into neuronal activity. Remarkably, the range of temperatures that these channels respond to covers the entire perceived temperature spectrum, from warm to painfully hot, and from pleasingly cool to excruciatingly cold. Moreover, many of these channels are receptors for ligands that elicit distinct psychophysical sensations, such as the heat associated with capsaicin and the cold felt with menthol.

Menthol modulates the TRPM8 receptor in a manner that produces the oral sensation of cooling. That is why menthol-containing natural products, such as mint extracts, tend to yield a cooling effect in the oral cavity. For this same reason, menthol is often used as an ingredient in carious non-extracted flavorings, where there is a desire to impart a cooling effect. Even so, menthol can have a bitter or astringent taste, to which some users may object. Thus, it can be desirable to develop strategies to enhance the cooling effect of menthol, thereby allowing for a reduction in the concentration of menthol and a concomitant reduction in undesirable astringency or bitterness.

Certain synthetic compounds may be used to the enhance cooling effect. These may be used alone or in combination with menthol. But such compounds often impart certain off tastes similar to menthol. Thus, there is a continuing need to discover new compositions that enhance the cooling effect of menthol in food, beverage, and oral care products without leading to the perception of bitterness or astringency.

### SUMMARY

The present disclosure relates to the discovery that certain compositions exhibit a unexpected effectiveness at enhancing a cooling sensation, such as the cooling sensation of menthol, in food, beverage, or oral care products.

The disclosure shows coolness-modifying compositions, the compositions comprising a coolness-enhancing compound selected from the group consisting of (E/Z)-2-methyl-2-butenal, (E/Z)-2-isopropyl-5-methyl-2-hexenal, and any combination thereof. In some embodiments, the coolness-modifying composition further comprises a high-intensity sweetener. In some embodiments, the high-intensity sweetener is sucralose, sodium saccharin, or a combination thereof. In some embodiments, the composition comprises a flavoring, such as a mint flavoring.

In a first aspect, the claimed invention provides uses of (E/Z)-2-methyl-2-butenal to enhance a cooling sensation of a flavored product. In some embodiments, the flavored product comprises a cooling compound, such as menthol or cubebol. In some embodiments, the flavored product is a food product or a beverage product. In some other embodiments, the flavored product is an oral care product, such as a toothpaste, a mouthwash, a dentifrice, a chewing gum, or a tooth-whitening composition.

In a second aspect, the claimed invention provides uses of (E/Z)-2-isopropyl-5-methyl-2-hexenal to enhance a cooling sensation of a flavored product. In some embodiments, the flavored product comprises a cooling compound, such as menthol or cubebol. In some embodiments, the flavored product is a food product or a beverage product. In some other embodiments, the flavored product is an oral care product, such as a toothpaste, a mouthwash, a dentifrice, a chewing gum, or a tooth-whitening composition.

The present disclosure thus shows methods of enhancing a cooling sensation of a cooling compound in a flavored product, comprising combining a cooling compound (for example, menthol, cubebol, or a combination thereof) and (E/Z)-2-methyl-2-butenal in a flavored product. In some embodiments, the flavored product is a food product or a beverage product. In some other embodiments, the flavored product is an oral care product, such as a toothpaste, a mouthwash, a dentifrice, a chewing gum, or a tooth-whitening composition.

In a further the present disclosure also shows methods of enhancing a cooling sensation of a cooling compound in a flavored product, comprising combining a cooling compound (for example, menthol, cubebol, or a combination thereof) and (E/Z)-2-isopropyl-5-methyl-2-hexenal in a flavored product. In some embodiments, the flavored product is a food product or a beverage product. In some other embodiments, the flavored product is an oral care product, such as a toothpaste, a mouthwash, a dentifrice, a chewing gum, or a tooth-whitening composition.

Furthermore, the present disclosure shows uses of the coolness-modifying composition mentioned above to enhance a cooling sensation of a flavored product. The flavored product comprises a cooling compound (for example, menthol, cubebol, or a combination thereof). In some embodiments, the flavored product is a food product or a beverage product. In some other embodiments, the flavored product is an oral care product, such as a toothpaste, a mouthwash, a dentifrice, a chewing gum, or a tooth-whitening composition.

In other words, methods enhancing a cooling sensation of a flavored products are shown, the method comprising combining the coolness-modifying composition mentioned above with a cooling compound (for example, menthol, cubebol, or a combination thereof) in a flavored product. In some embodiments, the flavored product is a food product or a beverage product. In some other embodiments, the flavored product is an oral care product, such as a toothpaste, a mouthwash, a dentifrice, a chewing gum, or a teeth-whitening product.

The present disclosure also shows flavored products comprising a coolness-modifying composition as mentioned above and a cooling compound (for example, menthol, cubebol, or a combination thereof). In some embodiments, the flavored product is a food product or a beverage product. In some other embodiments, the flavored product is an oral care product, such as a toothpaste, a mouthwash, a dentifrice, a chewing gum, or a teeth-whitening product.

The present disclosure provides uses of (E/Z)-2-methyl-2-butenal to enhance a cooling sensation of a body care product. The body care product comprises a cooling compound, such as menthol or cubebol. In some embodiments, the body care product is a shampoo, a body wash, a soap, an exfoliating scrub, a moisturizer, a facial serum, an antiperspirant, a deodorant, a body lotion, a body oil, a sunscreen, or a bronzer.

The present disclosure also provides uses of (E/Z)-2-isopropyl-5-methyl-2-hexenal to enhance a cooling sensation of a body care product. The body care product comprises a cooling compound, such as menthol or cubebol. In some embodiments, the body care product is a shampoo, a body wash, a soap, an exfoliating scrub, a moisturizer, a facial serum, an antiperspirant, a deodorant, a body lotion, a body oil, a sunscreen, or a bronzer.

In a further aspect, the disclosure shows methods of enhancing a cooling sensation of a cooling compound in a body care product, comprising combining a cooling compound (for example, menthol, cubebol, or a combination thereof) and (E/Z)-2-methyl-2-butenal in a body care product. In some embodiments, the body care product is a shampoo, a body wash, a soap, an exfoliating scrub, a moisturizer, a facial serum, an antiperspirant, a deodorant, a body lotion, a body oil, a sunscreen, or a bronzer.

Similarly, the disclosure also shows methods of enhancing a cooling sensation of a cooling compound in a body care product, comprising combining a cooling compound (for example, menthol, cubebol, or a combination thereof) and (E/Z)-2-isopropyl-5-methyl-2-hexenal in a body care product. In some embodiments, the body care product is a shampoo, a body wash, a soap, an exfoliating scrub, a moisturizer, a facial serum, an antiperspirant, a deodorant, a body lotion, a body oil, a sunscreen, or a bronzer.

The disclosure likewise shows uses of the coolness-modifying composition mentioned above to enhance a cooling sensation of a body care product. The body care product comprises a cooling compound (for example, menthol, cubebol, or a combination thereof). In some embodiments, the body care product is a shampoo, a body wash, a soap, an exfoliating scrub, a moisturizer, a facial serum, an antiperspirant, a deodorant, a body lotion, a body oil, a sunscreen, or a bronzer.

It also shows methods enhancing a cooling sensation of a body care product, the method comprising combining the coolness-modifying composition mentioned above with a cooling compound (for example, menthol, cubebol, or a combination thereof) in a body care product. In some embodiments, the body care product is a shampoo, a body wash, a soap, an exfoliating scrub, a moisturizer, a facial serum, an antiperspirant, a deodorant, a body lotion, a body oil, a sunscreen, or a bronzer.

Finally, the disclosure shows body care products comprising a coolness-modifying composition of the first aspect and a cooling compound (for example, menthol, cubebol, or a combination thereof). In some embodiments, the body care product is a shampoo, a body wash, a soap, an exfoliating scrub, a moisturizer, a facial serum, an antiperspirant, a deodorant, a body lotion, a body oil, a sunscreen, or a bronzer.

Further aspects, and embodiments thereof, are set forth below in the Detailed Description, the Drawings, the Abstract, and the Claims.

### DETAILED DESCRIPTION

The following Detailed Description sets forth various aspects and embodiments provided herein. The description is to be read from the perspective of the person of ordinary skill in the relevant art. Therefore, information that is well known to such ordinarily skilled artisans is not necessarily included.

### Definitions

The following terms and phrases have the meanings indicated below, unless otherwise provided herein. This disclosure may employ other terms and phrases not expressly defined herein. Such other terms and phrases have the meanings that they would possess within the context of this disclosure to those of ordinary skill in the art. In some instances, a term or phrase may be defined in the singular or plural. In such instances, it is understood that any term in the singular may include its plural counterpart and vice versa, unless expressly indicated to the contrary

A "sweetener", "sweet flavoring agent", "sweet flavor entity", or "sweet compound" herein refers to a compound or ingestibly acceptable salt thereof that elicits a detectable sweet flavor in a subject, e.g., a compound that activates a T1R2/T1R3 receptor in vitro.

A "cooling sensation" refers to a sensation produced by a ingestible article in a human of a reduction in ambient temperature, generally through modulation of the TRPM8 receptor.

A "cooling compound" refers to a compound that modulates the TRPM8 receptor. In some embodiments of any of the embodiments set forth herein, the cooling compound is menthol. Note, as used herein, "menthol" refers to (-)-menthol. In some embodiments of any of the embodiments set forth herein, the cooling compound is cubebol. Non-limiting examples of cooling compounds include N-ethyl-p-menthyl-3-carboxiamide, isopulegol, 3-(L-menthoxy) propane-1,2-diol, cis/trans-p-menthane-3,8-diol, 3-(L-menthoxy)-2-methylpropane-1,2-diol, 2-[2-(p-menthan-3-yloxy)ethoxy]ethanol, menthoxyethanol, (1R,2R,4R)-1-(2-hydroxy-4-methylcyclohexyl)ethenone, (1R,2R,5R)-N-ethyl-5-methyl-2-(prop-1-en-2-yl)-cyclohexanecarboxamide, N-(3-hydroxy-4-methoxyphenyl)-2-isopropyl-5,5-dimethyl-cyclohexanecarboxamide, N-[4-(cyanomethyl)phenyl]-2-isopropyl-5,5-dimethyl-cyclohexanecarboxamide, N-(2-Hydroxy-2-phenylethyl)-2-isopropyl-5,5-dimethyl-cyclohexane-1-carboxamide, L-menthol lactate, (-)-menthyl ethylene glycol carbonate, (-)-menthone 1,2-glycerol ketal, D/L-menthone 1,2-glycerol ketal, (-)-menthyl 1-propylene glycol carbonate, (-)-menthyl 2-propylene glycol carbonate, D/L-menthyl 1-propylene glycol carbonate, D/L-menthyl 2-propylene glycol carbonate, (1R,2S,5R)-N-(4-methoxyphenyl)-5-methyl-2-(1-methylethyl)cyclohexanecarboxamide, menthyl ethylamido oxalate, (E)-3-benzo[1,3]dioxol-5-yl-N,N-diphenyl-2-propenamide, 3,4-methylenedioxycinnamic acid, N,N-diphenylamide, N,N-dimethyl (-)-menthyl succinimide, (1R,2S,5R)-N-(4-(carbamoylmethyl)phenyl)-menthylcarboxamide, (-)-menthyl pyrrolidone carboxylate, L-phenylephrine p-menthane carboxamide, (1R,2S,5R)-N-(4-(cyanomethyl)-phenyl)menthylcarboxamide, (1R,2S,5R)-N-(2-(pyridin-2-yl)ethyl)menthylcarboxamide, 6-isopropyl-3,9-dimethyl-1,4-dioxaspiro[4.5]decan-2-one, (1R,2R,4S)-dihydroumbellulol, N-ethyl-p-menthane-3-carboxamide, 2-isopropyl-N,2,3-trimethylbutyramide, ethyl 3-(p-menthane-3-carboxamido)acetate, N-ethyl-2,2-diisopropylbutanamide, N-(1,1-dimethyl-2-hydroxyethyl)-2,2-dimethylbutanamide, N-cyclopropyl-5-methyl-2-isopropyl-cyclohexanecarbonecarboxamide, (-)-menthyl acetoacetate, (-)-menthyl succinate, (-)-menthyl (S)-3-hydroxybutyrate, (-)-menthyl glutarate, 1-[2-hydroxyphenyl]-4-[2-nitrophenyl]-1,2,3,6-tetrahydropyrimidine-2-one, di-(-)-menthyl glutarate, and N-(2-hydroxyethyl)-2,3-dimethyl-2-isopropylbutanamide.

As used herein, the singular forms "a," "an," and "the" include plural referents unless the context clearly dictates otherwise. For example, reference to "a substituent" encompasses a single substituent as well as two or more substituents, and the like.

As used herein, "for example," "for instance," "such as," or "including" are meant to introduce examples that further clarify more general subject matter. Unless otherwise expressly indicated, such examples are provided only as an aid for understanding embodiments illustrated in the present disclosure, and are not meant to be limiting in any fashion. Nor do these phrases indicate any kind of preference for the disclosed embodiment.

As used herein, "comprise" or "comprises" or "comprising" or "comprised of" refer to groups that are open, meaning that the group can include additional members in addition to those expressly recited. For example, the phrase, "comprises A" means that A must be present, but that other members can be present too. The terms "include," "have," and "composed of" and their grammatical variants have the same meaning. In contrast, "consist of" or "consists of" or "consisting of" refer to groups that are closed. For example, the phrase "consists of A" means that A and only A is present.

As used herein, "E/Z" indicated that a compound having E-Z stereochemistry can exist as the E-configuration, the Z-configuration, or as any mixture of compounds in the E-configuration and Z-configuration.

As used herein, "optionally" means that the subsequently described event(s) may or may not occur. In some embodiments, the optional event does not occur. In some other embodiments, the optional event does occur one or more times.

As used herein, "or" is to be given its broadest reasonable interpretation, and is not to be limited to an either/or construction. Thus, the phrase "comprising A or B" means that A can be present and not B, or that B is present and not A, or that A and B are both present. Further, if A, for example, defines a class that can have multiple members, e.g., A₁ and A₂, then one or more members of the class can be present concurrently.

Other terms are defined in other portions of this description, even though not included in this subsection.

### Coolness-Enhancing Compounds

In certain aspects, the disclosure provides uses of (E/Z)-2-methyl-2-butenal to enhance a cooling sensation of a flavored product. In some embodiments, the flavored product comprises a cooling compound, such as menthol or cubebol. In a related aspect, the disclosure provides methods of enhancing a cooling sensation of a cooling compound (such as menthol or cubebol) in a flavored product, comprising combining the cooling compound and (E/Z)-2-methyl-2-butenal in a flavored product. In some embodiments, the flavored product is a food product or a beverage product. In some other embodiments, the flavored product is an oral care product, such as a toothpaste, a mouthwash, a dentifrice, a chewing gum, or a tooth-whitening composition.

In certain other aspects aspect, the disclosure provides uses of (E/Z)-2-methyl-2-butenal to enhance a cooling sensation of a body care product. In some embodiments, the body care product comprises a cooling compound, such as menthol or cubebol. In a related aspect, the disclosure provides methods of enhancing a cooling sensation of a cooling compound (such as menthol or cubebol) in a body care product, comprising combining the cooling compound and (E/Z)-2-methyl-2-butenal in the body care product. In some embodiments, the body care product is a shampoo, a body wash, a soap, an exfoliating scrub, a moisturizer, a facial serum, an antiperspirant, a deodorant, a body lotion, a body oil, a sunscreen, or a bronzer.

As used herein, "(E/Z)-2-methyl-2-butenal" refers to the E- or Z-configuration of 2-methyl-2-butenal, or any mixture thereof. In some embodiments, the (E/Z)-2-methyl-2-butenal is (E)-2-methyl-2-butenal. In some other embodiments, the (E/Z)-2-methyl-2-butenal is (Z)-2-methyl-2-butenal. In some embodiments, the (E/Z)-2-methyl-2-butenal is a mixture of (E)-2-methyl-2-butenal and (Z)-2-methyl-2-butenal. Suitable w/w ratios of the E-configuration to the Z-configuration range from 100:1 to 1:100, or from 50:1 to 1:50, or from 20:1 to 1:20, or from 10:1 to 1:10, or from 5:1 to 1:5, or from 3:1 to 1:3, or from 2:1 to 1:2. In some embodiments, such mixtures comprise a greater amount of the E-configuration relative to the Z-configuration. In some such embodiments, the w/w ratios of the E-configuration to the Z-configuration range from 3:2 to 100:1, or from 3:2 to 50:1, or from 3:2 to 25:1, or from 3:2 to 10:1, or from 3:2 to 5:1, or from 3:2 to 4:1, or from 3:2 to 3:1, or from 3:2 to 2:1.

In aspects related to the use or introduction to a flavored product, the flavored product can be any suitable food product, beverage product, or oral care product, each of which are described in further detail below. As set forth in greater detail below, the flavored product can include any variety of other components, including flavorings, natural and artificial sweeteners, and the like.

In aspects related to the use or introduction to a body care product, the body care product can be any suitable product suitable for use on hair, skin, and the like, which are described in further detail below. As set forth in greater detail below, the body care product can include any variety of other components, such as components typically included in body care products.

The (E/Z)-2-methyl-2-butenal can be present at any suitable concentration in the flavored product or body care product. In some embodiments, the concentration of (E/Z)-2-methyl-2-butenal in the flavored product or body care product ranges from 1 ppm to 1000 ppm, or from 5 ppm to 500 ppm, or from 10 ppm to 300 ppm, or from 20 ppm to 200 ppm, based on the total weight of the product.

In some embodiments, the flavored product further comprises a high-intensity sweetener. In some such embodiments, the high-intensity sweetener is sodium saccharin or sucralose. In some embodiments, the high-intensity sweetener is sodium saccharin. The sodium saccharin can be present in any suitable concentration. In some embodiments, the concentration of sodium saccharin ranges from 10 ppm to 10000 ppm, or from 50 ppm to 5000 ppm, or from 100 ppm to 3000 ppm, based on the total weight of the product.

In some embodiments, the flavored product or body care product comprises menthol, i.e., (-)-menthol. The menthol can be present in any suitable concentration. In some embodiments, the menthol is present at a concentration ranging from 10 ppm to 20000 ppm, or from 50 ppm to 10000 ppm, or from 100 ppm to 7000 ppm, or from 500 ppm to 5000 ppm, based on the total weight of the product.

In some embodiments, the flavored product or body care product comprises menthol, The menthol can be present in the flavored product or body care product at a relative concentration (w/w) to the (E/Z)-2-methyl-2-butenal in any suitable ratio. In some embodiments, the w/w ratio of menthol to (E/Z)-2-methyl-2-butenal in the flavored product ranges from 1:1 to 200:1, or from 5:1 to 100:1.

In some embodiments, the flavored product or body care product comprises cubebol, which is also referred to as (3S,3aR,3bR,4S,7R,7aR)-octahydro-3,7-dimethyl-4-(1-methylethyl)-1H-cyclopenta[1.3]cyclopropa[1.2]benzen-3-ol. Cubebol can be present at any suitable concentration in the flavored product or body care product. In some embodiments, the concentration of cubebol in the flavored product or body care product ranges from 1 ppm to 1000 ppm, or from 5 ppm to 500 ppm, or from 10 ppm to 300 ppm, or from 20 ppm to 200 ppm, based on the total weight of the product.

Other compounds and additives can be included in the flavored product or body care product, as set forth in greater detail below. For example, in some embodiments, the flavored product or body care product comprises one or both of (E/Z)-2-isopropyl-5-methyl-2-hexenal or any other cooling compounds, such as any of those set forth above.

In certain aspects, the disclosure provides uses of (E/Z)-2-isopropyl-5-methyl-2-hexenal to enhance a cooling sensation of a flavored product. In some embodiments, the flavored product comprises a cooling compound, such as menthol or cubebol. In a related aspect, the disclosure provides methods of enhancing a cooling sensation of a cooling compound (such as menthol or cubebol) in a flavored product, comprising combining the cooling compound and (E/Z)-2-isopropyl-5-methyl-2-hexenal in a flavored product. In some embodiments, the flavored product is a food product or a beverage product. In some other embodiments, the flavored product is an oral care product, such as a toothpaste, a mouthwash, a dentifrice, a chewing gum, or a tooth-whitening composition.

In certain other aspects aspect, the disclosure provides uses of (E/Z)-2-isopropyl-5-methyl-2-hexenal to enhance a cooling sensation of a body care product. In some embodiments, the body care product comprises a cooling compound, such as menthol or cubebol. In a related aspect, the disclosure provides methods of enhancing a cooling sensation of a cooling compound (such as menthol or cubebol) in a body care product, comprising combining the cooling compound and (E/Z)-2-isopropyl-5-methyl-2-hexenal in the body care product. In some embodiments, the body care product is a shampoo, a body wash, a soap, an exfoliating scrub, a moisturizer, a facial serum, an antiperspirant, a deodorant, a body lotion, a body oil, a sunscreen, or a bronzer.

As used herein, "(E/Z)-2-isopropyl-5-methyl-2-hexenal" refers to the E- or Z-configuration of 2-isopropyl-5-methyl-2-hexenal, or any mixture thereof. In some embodiments, the (E/Z)-2-isopropyl-5-methyl-2-hexenal is (E)-2-isopropyl-5-methyl-2-hexenal. In some other embodiments, the (E/Z)-2-isopropyl-5-methyl-2-hexenal is (Z)-2-isopropyl-5-methyl-2-hexenal. In some embodiments, the (E/Z)-2-isopropyl-5-methyl-2-hexenal is a mixture of (E)-2-isopropyl-5-methyl-2-hexenal and (Z)-2-isopropyl-5-methyl-2-hexenal. Suitable w/w ratios of the E-configuration to the Z-configuration range from 100:1 to 1:100, or from 50:1 to 1:50, or from 20:1 to 1:20, or from 10:1 to 1:10, or from 5:1 to 1:5, or from 3:1 to 1:3, or from 2:1 to 1:2. In some embodiments, such mixtures comprise a greater amount of the E-configuration relative to the Z-configuration. In some such embodiments, the w/w ratios of the E-configuration to the Z-configuration range from 3:2 to 100:1, or from 3:2 to 50:1, or from 3:2 to 25:1, or from 3:2 to 10:1, or from 3:2 to 5:1, or from 3:2 to 4:1, or from 3:2 to 3:1, or from 3:2 to 2:1.

In aspects related to the use or introduction to a flavored product, the flavored product can be any suitable food product, beverage product, or oral care product, each of which are described in further detail below. As set forth in greater detail below, the flavored product can include any variety of other components, including flavorings, natural and artificial sweeteners, and the like.

In aspects related to the use or introduction to a body care product, the body care product can be any suitable product suitable for use on hair, skin, and the like, which are described in further detail below. As set forth in greater detail below, the body care product can include any variety of other components, such as components typically included in body care products.

The (E/Z)-2-isopropyl-5-methyl-2-hexenal can be present at any suitable concentration in the flavored product or body care product. In some embodiments, the concentration of (E/Z)-2-isopropyl-5-methyl-2-hexenal in the flavored product ranges from 1 ppm to 1000 ppm, or from 5 ppm to 500 ppm, or from 10 ppm to 300 ppm, or from 20 ppm to 200 ppm, based on the total weight of the product.

In some embodiments, the flavored product comprises a high-intensity sweetener. In some such embodiments, the high-intensity sweetener is sodium saccharin or sucralose. In some embodiments, the high-intensity sweetener is sodium saccharin. The sodium saccharin can be present in any suitable concentration. In some embodiments, the concentration of sodium saccharin ranges from 10 ppm to 10000 ppm, or from 50 ppm to 5000 ppm, or from 100 ppm to 3000 ppm, based on the total weight of the product.

In some embodiments, the flavored product comprises menthol, i.e., (-)-menthol. The menthol can be present in any suitable concentration. In some embodiments, the menthol is present at a concentration ranging from 10 ppm to 20000 ppm, or from 50 ppm to 10000 ppm, or from 100 ppm to 7000 ppm, or from 500 ppm to 5000 ppm, based on the total weight of the product.

In some embodiments, the flavored product or body care product comprises menthol. In such embodiments, the menthol can be present in the flavored product or body care product at a relative concentration (w/w) to the (E/Z)-2-isopropyl-5-methyl-2-hexenal in any suitable ratio. In some embodiments, the w/w ratio of menthol to (E/Z)-2-isopropyl-5-methyl-2-hexenal in the flavored product ranges from 1:1 to 200:1, or from 5:1 to 100:1.

In some embodiments, the flavored product or body care product comprises cubebol, which is also referred to as (3S,3aR,3bR,4S,7R,7aR)-octahydro-3,7-dimethyl-4-(1-methylethyl)-1H-cyclopenta[1.3]cyclopropa[1.2]benzen-3-ol. Cubebol can be present at any suitable concentration in the flavored product. In some embodiments, the concentration of cubebol in the flavored product ranges from 1 ppm to 1000 ppm, or from 5 ppm to 500 ppm, or from 10 ppm to 300 ppm, or from 20 ppm to 200 ppm, based on the total weight of the product.

Other compounds and additives can be included in the flavored product or body care product, as set forth in greater detail below. For example, in some embodiments, the flavored product comprises one or both of (E/Z)-2-methyl-2-butenal or any other cooling compounds, such as any of those set forth above.

### Coolness-Modifying Compositions

The coolness-enhancing compounds (according to any of the embodiments set forth above) can be included in any suitable compositions for purposes of delivery or introduction into a flavored product or body care product.

In at least a first aspect, the disclosure provides coolness-modifying compositions, the compositions comprising a coolness-enhancing compound selected from the group consisting of (E/Z)-2-methyl-2-butenal, (E/Z)-2-isopropyl-5-methyl-2-hexenal, and any combination thereof.

The coolness-modifying compositions may comprise any combination of the coolness-enhancing compounds. In some further such embodiments, the coolness-enhancing composition comprises a combination of (E/Z)-2-methyl-2-butenal (according to any of the embodiments set forth above), and (E/Z)-2-isopropyl-5-methyl-2-hexenal (according to any of the embodiments set forth above).

The coolness-modifying compositions may comprise any combination of the coolness-enhancing compounds. In some embodiments, the coolness-enhancing composition comprises (E/Z)-2-methyl-2-butenal (according to any of the embodiments set forth above). In some other such embodiments, the coolness-enhancing composition comprises a combination of (E/Z)-2-isopropyl-5-methyl-2-hexenal (according to any of the embodiments set forth above). In some other such embodiments, the coolness-enhancing composition comprises a combination of (E/Z)-2-methyl-2-butenal (according to any of the embodiments set forth above) and (E/Z)-2-isopropyl-5-methyl-2-hexenal (according to any of the embodiments set forth above).

In the preceding embodiments, where the coolness-modifying composition comprises a combination of coolness-enhancing compounds, the coolness-enhancing compounds can be present in any suitable relative amounts. In some embodiments, where the coolness-modifying composition comprises a combination of coolness-enhancing compounds, the ratio (w/w) of any two coolness-enhancing compounds ranges from 1:20 to 20:1, or from 1:10 to 10:1, or from 1:5 to 5:1, or from 1:3 to 3:1, or from 1:2 to 2:1, or from 2:3 to 3:2.

Further, the coolness-enhancing compounds can be present in the coolness-modifying composition in any suitable concentration. In embodiments, where the coolness-modifying composition is a flavored product suitable for human ingestion, the coolness-enhancing compounds are present at a concentration ranging from 1 ppm to 1000 ppm, or from 5 ppm to 500 ppm, or from 10 ppm to 300 ppm, or from 20 ppm to 200 ppm.

The coolness-modifying compositions may comprise any number of additional ingredients, such as ingredients typically used as additives and excipients in food products, beverage products, oral care products, such as toothpaste, mouthwash, and chewing gum, and body care products.

In some embodiments, the coolness-modifying compositions comprise menthol, namely (-)-menthol. The menthol can be present in any suitable concentration. In some embodiments, where the coolness-modifying composition is a flavored product suitable for human ingestion, the menthol is present at a concentration ranging from 10 ppm to 20000 ppm, or from 50 ppm to 10000 ppm, or from 100 ppm to 7000 ppm, or from 500 ppm to 5000 ppm, based on the total weight of the composition. In some embodiments, where the coolness-modifying composition is a body care product suitable for application to human skin or hair, the menthol is present at a concentration ranging from 10 ppm to 20000 ppm, or from 50 ppm to 10000 ppm, or from 100 ppm to 7000 ppm, or from 500 ppm to 5000 ppm, based on the total weight of the composition. The menthol can be present at a relative concentration (w/w) to the coolness-enhancing compounds in any suitable ratio. In some embodiments, the w/w ratio of menthol to the coolness-enhancing compounds in the coolness-modifying composition ranges from 1:1 to 200:1, or from 5:1 to 100:1.

In some embodiments, the coolness-modifying compositions comprise cubebol. Cubebol can be present at any suitable concentration in the flavored product or body care product. In some embodiments, the concentration of cubebol in the flavored product or body care product ranges from 1 ppm to 1000 ppm, or from 5 ppm to 500 ppm, or from 10 ppm to 300 ppm, or from 20 ppm to 200 ppm, based on the total weight of the composition.

In some embodiments, the coolness-modifying compositions comprise other cooling agents, such as N-ethyl-p-menthyl-3-carboxamide. N-Ethyl-p-menthyl-3-carboxamide can be present in any suitable concentration. In some embodiments, where the coolness-modifying composition is a flavored product suitable for human ingestion, the N-ethyl-p-menthyl-3-carboxamide is present at a concentration ranging from 1 ppm to 20000 ppm, or from 5 ppm to 10000 ppm, or from 10 ppm to 7000 ppm, or from 50 ppm to 5000 ppm, based on the total weight of the composition. In some embodiments, where the coolness-modifying composition is a body care product suitable for application to human skin or hair, the N-ethyl-p-menthyl-3-carboxamide is present at a concentration ranging from 1 ppm to 20000 ppm, or from 5 ppm to 10000 ppm, or from 10 ppm to 7000 ppm, or from 50 ppm to 5000 ppm, based on the total weight of the composition. The N-ethyl-p-menthyl-3-carboxamide can be present at a relative concentration (w/w) to the coolness-enhancing compounds in any suitable ratio. In some embodiments, the w/w ratio of N-ethyl-p-menthyl-3-carboxamide to the coolness-enhancing compounds in the coolness-modifying composition ranges from 1:1 to 200:1, or from 5:1 to 100:1.

In some embodiments, the coolness-modifying compositions comprise a cooling compound. In some embodiments, the cooling compound is menthol. In some embodiments, the cooling compound is cubebol. In some embodiments, the cooling compound is N-ethyl-p-menthyl-3-carboxamide. In some embodiments, the cooling compound is: menthol, cubebol, N-ethyl-p-menthyl-3-carboxamide, isopulegol, 3-(L-menthoxy) propane-1,2-diol, cis/trans-p-menthane-3,8-diol, 3-(L-menthoxy)-2-methylpropane-1,2-diol, 2-[2-(p-menthan-3-yloxy)ethoxy]ethanol, menthoxyethanol, (1R,2R,4R)-1-(2-hydroxy-4-methylcyclohexyl)-ethenone, (1R,2R,5R)-N-ethyl-5-methyl-2-(prop-1-en-2-yl)-cyclohexanecarboxamide, N-(3-hydroxy-4-methoxyphenyl)-2-isopropyl-5,5-dimethyl-cyclohexanecarboxamide, N-[4-(cyanomethyl)phenyl]-2-isopropyl-5,5-dimethyl-cyclohexanecarboxamide, N-(2-Hydroxy-2-phenylethyl)-2-isopropyl-5,5-dimethyl-cyclohexane-1-carboxamide, L-menthol lactate, (-)-menthyl ethylene glycol carbonate, (-)-menthone 1,2-glycerol ketal, D/L-menthone 1,2-glycerol ketal, (-)-menthyl 1-propylene glycol carbonate, (-)-menthyl 2-propylene glycol carbonate, D/L-menthyl 1-propylene glycol carbonate, D/L-menthyl 2-propylene glycol carbonate, (1R,2S,5R)-N-(4-methoxyphenyl)-5-methyl-2-(L-methylethyl)cyclohexanecarboxamide, menthyl ethylamido oxalate, (E)-3-benzo[1,3]dioxol-5-yl-N,N-diphenyl-2-propenamide, 3,4-methylenedioxycinnamic acid, N,N-diphenylamide, N,N-dimethyl (-)-menthyl succinimide, (1R,2S,5R)-N-(4-(carbamoylmethyl)phenyl)-menthylcarboxamide, (-)-menthyl pyrrolidone carboxylate, L-phenylephrine p-menthane carboxamide, (1R,2S,SR)-N-(4-(cyanomethyl)-phenyl)menthylcarboxamide, (1R,2S,5R)-N-(2-(pyridin-2-yl)ethyl)menthylcarboxamide, 6-isopropyl-3,9-dimethyl-1,4-dioxaspiro[4.5]decan-2-one, (1R,2R,4S)-dihydroumbellulol, N-ethyl-p-menthane-3-carboxamide, 2-isopropyl-N,2,3-trimethylbutyramide, ethyl 3-(p-menthane-3-carboxamido)acetate, N-ethyl-2,2-diisopropylbutanamide, N-(1,1-dimethyl-2-hydroxyethyl)-2,2-dimethylbutanamide, N-cyclopropyl-5-methyl-2-isopropyl-cyclohexanecarbonecarboxamide, (-)-menthyl acetoacetate, (-)-menthyl succinate, (-)-menthyl (S)-3-hydroxybutyrate, (-)-menthyl glutarate, 1-[2-hydroxyphenyl]-4-[2-nitrophenyl]-1,2,3,6-tetrahydropyrimidine-2-one, di-(-)-menthyl glutarate, N-(2-hydroxyethyl)-2,3-dimethyl-2-isopropylbutanamide, or any combination thereof.

### Coolness-Modifying Compositions Suitable for Flavored Products

In some embodiments of any of the foregoing embodiments, the coolness-modifying compositions comprises a sweetener, such as a low-calorie, zero-calorie, or high-intensity sweetener. The term "high-intensity sweetener" refers to sweeteners that are sweeter than sucrose, such as at least 10 times, or at least 20 times, or at least 50 times, or at least 100 times, or at least 250 times, or at least 500 times, or at least 750 times, or at least 1000 times, or at least 2500 times, or at least 5000 times, or at least 7500 times, or at least 10000 times, sweeter than sucrose. In this context, to say that a sweetener is x times sweeter than sucrose means that a concentration of 1/x by weight relative to sucrose in aqueous solution of said sweetener yields an equivalent sweetness as sucrose. Examples of suitable sweeteners are set forth in greater detail below. In some embodiments, the sweetener is sucralose, which refers to 1,6-dichloro-1,6-dideoxy-β-D-fructofuranosyl-4-chloro-4-deoxy-α-D-galactopyranoside. In some embodiments, the sweetener is sodium saccharin. The sodium saccharin can be present in any suitable concentration. In some embodiments, the concentration of sodium saccharin ranges from 10 ppm to 10000 ppm, or from 50 ppm to 5000 ppm, or from 100 ppm to 3000 ppm. The sodium saccharin can have any suitable relative concentration to the coolness-enhancing compounds. In some embodiments, the ratio (w/w) of the sodium saccharin relative to the coolness-enhancing compounds in the coolness-modifying composition ranges from 1:1 to 1000:1, or from 5:1 to 500:1.

In some embodiments, the coolness-modifying composition comprises glucosylated natural steviol glycosides. As used herein, the term "natural steviol glycosides" refers to steviol glycosides naturally found in the plants of the species *Stevia rebaudiana*, *Stevia phlebophylla*, or *Rubus chingii.* Such compounds are glycosides of the diterpene, steviol. Some non-limiting examples include stevioside, rebaudioside A, rebaudioside C, dulcoside A, and the like. As used herein, the term "glucosylated natural steviol glycosides" refers to compounds obtained by the enzymatic glucosylation of natural steviol glycosides to increase the degree of glucosylation. As used herein, the term "GSG" is used interchangeably with "glucosylated natural steviol glycoside."

In embodiments in which GSGs are present, any suitable GSGs may be used. Thus, the GSGs can have any suitable degree of additional glucosylation, and ant suitable type of glucosylation. In some embodiments, the GSGs used in the coolness-modifying composition have a degree of glucosylation of at least 1.0, meaning that at least one mole of glucose has been enzymatically added to the natural steviol glycosides per mole of natural steviol glycoside. In some other embodiments, the GSGs used in the coolness-modifying composition have a degree of glucosylation of at least 1.1, or at least 1.2, or at least 1.3, or at least 1.4, or at least 1.5, or at least 1.6, or at least 1.7, or at least 1.8, or at least 1.9, or at least 2.0, or at least 2.1, or at least 2.2, or at least 2.3, or at least 2.4, or at least 2.5. The glucosylation can be of any suitable type. For example, in some embodiments, the glucose enzymatically added to the natural steviol glycosides is alpha-glucose, beta-glucose, or a combination thereof. In some embodiments, the glucose enzymatically added to the natural steviol glycosides is alpha-glucose. In some embodiments, the glucose enzymatically added to the natural steviol glycosides is beta-glucose. In some embodiments, the glucose enzymatically added to the natural steviol glycosides is a combination of alpha-glucose and beta-glucose. The glucose added by enzymatic glucosylation can be incorporated via any suitable connection. Typically, the added glucose units connect to other glucose glucose units already present on the natural steviol glycoside. In some embodiments, the enzymatically added glucose units are added via 1,6 linkages, meaning that the glucose units add via the 1-position of the added glucose unit and the 6-position of the glucose unit already present on the natural steviol glycoside. In some other embodiments, the enzymatically added glucose units are added via a 1,4 linkages. In some other embodiments, the enzymatically added glucose units are added via a 1,2 linkages.

In embodiments where GSGs are present, the GSGs can be present in any suitable concentration. In some embodiments, the GSGs are present in the coolness-modifying composition at a concentration ranging from 1 ppm to 100 ppm, or from 1 pm to 50 ppm, or from 1 ppm to 30 ppm, or from 1 ppm to 20 ppm, or from 1 ppm to 10 ppm.

In some embodiments, the coolness-modifying composition comprises 4-amino-5,6-dimethylthieno[2,3-d]pyrimidin-2(1H)-one, or any suitable salt thereof can be used. In some embodiments, the coolness-modifying compositions disclosed herein comprise the hydrochloride salt of 4-amino-5,6-dimethylthieno[2,3-d]pyrimidin-2(1H)-one.

In some embodiments of any of the foregoing embodiments, the coolness-modifying composition also comprises a lactone. Any suitable lactone or combination of lactones can be used. In some embodiments, the lactone comprises a lactone ring having from 3 to 8 members in the ring, such as a 3-membered lactone ring, a 4-membered lactone ring, a 5-membered lactone ring, a 6-membered lactone ring, a 7-membered lactone ring, or an 8-membered lactone ring.

In some embodiments, the lactone comprises a 5-membered lactone ring with or without an unsaturated bond (excluding the unsaturation in the oxo substituent on the ring), such as a carbon-carbon double bond. In some embodiments, the lactone comprises a 5-membered lactone ring including at least one unsaturated bond (for example, one carbon-carbon double bond). Non-limiting examples of lactones comprising a 5-membered lactone ring having at least one unsaturated bond include angelica lactone alpha, angelica lactone beta, mint lactone, 5,5-dimethyl-2(5H)-furanone, 5-ethyl-2(5H)-furanone, 5-pentyl-2(5H)-furanone, 5-hexyl-2(5H)-furanone, 5-pentyl-2(3H)-furanone, 4-methyl-3-pentyl-2(5H)-furanone, and 2(5H)-furanone. In some embodiments, the lactone comprises a 5-membered lactone ring without any unsaturated bonds. Non-limiting examples of such lactones include butyrolactone gamma, valerolactone gamma, hexalactone gamma, heptalactone gamma, octalactone gamma, undecalactone gamma, decalactone gamma, dihydrojasmone lactone, and dihydromintlactone (3,6-dimethyl-hexahydro-2(3H)-benzofuranone). In some embodiments, the lactone is dihydromintlactone.

In some embodiments, the lactone comprises a 6-membered lactone ring with or without an unsaturated bond. In some embodiments, the lactone comprises a 6-membered lactone ring including at least one unsaturated bond. Non-limiting examples of lactones comprising a 6-membered lactone ring having at least one unsaturated bond include δ-2-decenolactone, coumarin, and 6-methylcoumarin. In some other embodiments, the lactone includes a 6-membered lactone ring without any unsaturated bonds. Non-limiting examples of lactones including a 6-membered lactone ring without any unsaturated bonds include decalactone delta, dodecalactone delta, and cyclohexyl lactone.

For example, in some embodiments, the coolness-modifying composition comprises a caloric sugar, such as sucrose, glucose, fructose (e.g., in the form of high-fructose corn syrup), or any combination thereof. In some embodiments, the coolness-modifying composition comprises one or more rebaudiosides (such as rebaudioside A, rebaudioside D, rebaudioside E, rebaudioside M, or any combination thereof). In general, the concentrations of rebaudiosides (such as rebaudioside A) would be about 20 to 50 times higher than those set forth above for sucralose. In some embodiments, the coolness-modifying composition comprises one or more high-intensity artificial sweeteners, such as acefulfame potassium, aspartame, cyclamate, neotame, and the like. When any such artificial high-intensity sweeteners are present in the coolness-modifying composition, their concentration would be about the same as those set forth above for sucralose. In some other embodiments, the coolness-modifying compositions comprise one or more low-calorie carbohydrates or sugar alcohols, such as allulose, xylitol, erythritol, and the like. In some other embodiments, the coolness-modifying compositions comprise mogrosides, for example, as monk fruit juice or extract, or as one or more of mogroside III, mogroside IV, mogroside V, siamenoside I, isomogroside V, mogroside IV_{E}, isomogroside IV_{E}, isomogroside IV, mogroside III_{E}, 11-oxomogroside V, the 1,6-alpha isomer of siamenoside I, and any combinations thereof. Additional mogroside compounds that may be suitably included in the coolness-modifying compostiion are described in U.S. Patent Application Publication No. 2017/0119032.

Various other sweeteners may also be included in the coolness-modifying compositions. Non-limiting examples include D-psicose, L-ribose, D-tagatose, L-glucose, L-fucose, L-arbinose, D-turanose, D-leucrose, isomalt, lactitol, mannitol, sorbitol, maltodextrin, saccharin, alitame, cyclamic acid, tagatose, maltose, galactose, mannose, lactose, D-tryptophan, glycine, maltitol, lactitol, isomalt, hydrogenated starch hydrolyzate (HSH), chemically modified mogrosides (such as glucosylated mogrosides), carrelame and other guanidine-based sweeteners, honey, Jerusalem artichoke syrup, licorice root, luo han guo (fruit, powder, or extracts), lucuma (fruit, powder, or extracts), maple sap (including, for example, sap extracted from *Acer saccharum, Acer nigrum, Acer rubrum, Acer saccharinum, Acer platanoides, Acer negundo, Acer macrophyllum, Acer grandidentatum, Acer glabrum*, *Acer mono*), maple syrup, maple sugar, walnut sap (including, for example, sap extracted from *Juglans cinerea, Juglans nigra, Juglans ailatifolia*, *Juglans regia*), birch sap (including, for example, sap extracted from *Betula papyrifera, Betula alleghaniensis*, *Betula lenta*, *Betula nigra, Betula populifolia, Betula pendula*), sycamore sap (such as, for example, sap extracted from *Platanus occidentalis*), ironwood sap (such as, for example, sap extracted from *Ostrya virginiana*), mascobado, molasses (such as, for example, blackstrap molasses), molasses sugar, monatin, monellin, cane sugar (also referred to as natural sugar, unrefined cane sugar, or sucrose), palm sugar, panocha, piloncillo, rapadura, raw sugar, rice syrup, sorghum, sorghum syrup, cassava syrup (also referred to as tapioca syrup), thaumatin, yacon root, malt syrup, barley malt syrup, barley malt powder, beet sugar, cane sugar, crystalline juice crystals, caramel, carbitol, carob syrup, castor sugar, hydrogenated starch hydrolates, hydrolyzed can juice, hydrolyzed starch, invert sugar, anethole, arabinogalactan, arrope, syrup, P-4000, acesulfame potassium (also referred to as acesulfame K or ace-K), alitame (also referred to as aclame), advantame, aspartame, baiyunoside, neotame, benzamide derivatives, bernadame, canderel, carrelame and other guanidine-based sweeteners, vegetable fiber, corn sugar, coupling sugars, curculin, cyclamates, cyclocarioside I, demerara, dextran, dextrin, diastatic malt, dulcin, sucrol, valzin, dulcoside A, dulcoside B, emulin, enoxolone, maltodextrin, saccharin, estragole, ethyl maltol, glucin, gluconic acid, glucono-lactone, glucosamine, glucoronic acid, glycerol, glycine, glycyphillin, glycyrrhizin, glycyrrhetic acid monoglucuronide, golden sugar, yellow sugar, golden syrup, granulated sugar, gynostemma, hernandulcin, isomerized liquid sugars, jallab, chicory root dietary fiber, kynurenine derivatives (including N'-formyl-kynurenine, N'-acetyl-kynurenine, 6-chloro-kynurenine), galactitol, litesse, ligicane, lycasin, lugduname, guanidine, falemum, mabinlin I, mabinlin II, maltol, maltisorb, maltodextrin, maltotriol, mannosamine, miraculin, mizuame, mogrosides (including, for example, mogroside IV, mogroside V, and neomogroside), mukurozioside, nano sugar, naringin dihydrochalcone, neohesperidine dihydrochalcone, nib sugar, nigerooligosaccharide, norbu, orgeat syrup, osladin, pekmez, pentadin, periandrin I, perillaldehyde, perillartine, petphyllum, phenylalanine, phlomisoside I, phlorodizin, phyllodulcin, polyglycitol syrups, polypodoside A, pterocaryoside A, pterocaryoside B, rebiana, refiners syrup, rub syrup, rubusoside, selligueain A, shugr, siamenoside I, siraitia grosvenorii, soybean oligosaccharide, Splenda, SRI oxime V, steviol glycoside, steviolbioside, stevioside, strogins 1, 2, and 4, sucronic acid, sucrononate, sugar, suosan, phloridzin, superaspartame, tetrasaccharide, threitol, treacle, trilobtain, tryptophan and derivatives (6-trifluoromethyl-tryptophan, 6-chloro-D-tryptophan), vanilla sugar, volemitol, birch syrup, aspartameacesulfame, assugrin, and combinations or blends of any two or more thereof.

The coolness-modifying compositions can, in certain embodiments, comprise any additional ingredients or combination of ingredients as are commonly used in food and beverage products, including, but not limited to:
acids, including, for example citric acid, phosphoric acid, ascorbic acid, sodium acid sulfate, lactic acid, or tartaric acid;
bitter ingredients, including, for example caffeine, quinine, green tea, catechins, polyphenols, green robusta coffee extract, green coffee extract, potassium chloride, menthol, or proteins (such as proteins and protein isolates derived from plants, algae, or fungi);
coloring agents, including, for example caramel color, Red #40, Yellow #5, Yellow #6, Blue #1, Red #3, purple carrot, black carrot juice, purple sweet potato, vegetable juice, fruit juice, beta carotene, turmeric curcumin, or titanium dioxide;
preservatives, including, for example sodium benzoate, potassium benzoate, potassium sorbate, sodium metabisulfate, sorbic acid, or benzoic acid;
antioxidants including, for example ascorbic acid, calcium disodium EDTA, alpha tocopherols, mixed tocopherols, rosemary extract, grape seed extract, resveratrol, or sodium hexametaphosphate;
vitamins or functional ingredients including, for example resveratrol, Co-Q10, omega 3 fatty acids, theanine, choline chloride (citocoline), fibersol, inulin (chicory root), taurine, panax ginseng extract, guanana extract, ginger extract, L-phenylalanine, L-carnitine, L-tartrate, D-glucoronolactone, inositol, bioflavonoids, Echinacea, ginko biloba, yerba mate, flax seed oil, garcinia cambogia rind extract, white tea extract, ribose, milk thistle extract, grape seed extract, pyrodixine HCl (vitamin B6), cyanoobalamin (vitamin B12), niacinamide (vitamin B3), biotin, calcium lactate, calcium pantothenate (pantothenic acid), calcium phosphate, calcium carbonate, chromium chloride, chromium polynicotinate, cupric sulfate, folic acid, ferric pyrophosphate, iron, magnesium lactate, magnesium carbonate, magnesium sulfate, monopotassium phosphate, monosodium phosphate, phosphorus, potassium iodide, potassium phosphate, riboflavin, sodium sulfate, sodium gluconate, sodium polyphosphate, sodium bicarbonate, thiamine mononitrate, vitamin D3, vitamin A palmitate, zinc gluconate, zinc lactate, or zinc sulphate;
clouding agents, including, for example ester gun, brominated vegetable oil (BVO), or sucrose acetate isobutyrate (SAIB);
buffers, including, for example sodium citrate, potassium citrate, or salt;
flavors, including, for example propylene glycol, ethyl alcohol, glycerine, gum Arabic (gum acacia), maltodextrin, modified corn starch, dextrose, natural flavor, natural flavor with other natural flavors (natural flavor WONF), natural and artificial flavors, artificial flavor, silicon dioxide, magnesium carbonate, or tricalcium phosphate; or
starches and stabilizers, including, for example pectin, xanthan gum, carboxylmethylcellulose (CMC), polysorbate 60, polysorbate 80, medium chain triglycerides, cellulose gel, cellulose gum, sodium caseinate, modified food starch, gum Arabic (gum acacia), inulin, or carrageenan.

The coolness-modifying compositions can have any suitable pH when dissolved or suspended in aqueous media, e.g., from lower pH to neutral pH. The lower and neutral pH includes, but is not limited to, a pH from 1.5 to 9.0, or from 2.5 to 8.5; from 3.0 to 8.0; from 3.5 to 7.5; and from 4.0 to 7.

The coolness-modifying compositions set forth according to any of the foregoing embodiments, also include, in certain embodiments, one or more additional flavor-modifying compounds, such as compounds that enhance sweetness (e.g., hesperetin, naringenin, etc.), compounds that block bitterness, compounds that enhance umami, compounds that reduce sourness or licorice taste, compounds that enhance saltiness, compounds that enhance a cooling effect, or any combinations of the foregoing.

In some embodiments, coolness-modifying compositions disclosed herein comprise one or more sweetness enhancing compounds. Such sweetness enhancing compounds include, but are not limited to, naturally derived compounds, such as hesperitin, naringenin, rhoifolin, glucosylated steviol glycosides, licorice-derived glucuronates, aromadendrin-3-O-acetate, or other like flavonols, or flavonoids, or synthetic compounds, such as any compounds set forth in U.S. Patent Nos. 8,541,421; 8,815,956; 9,834,544; 8,592,592; 8,877,922; 9,000,054; and 9,000,051, as well as U.S. Patent Application Publication No. 2017/0119032. Some suitable examples include: 3-((4-amino-2,2-dioxo-1H-benzo[c][1,2,6]thiadiazin-5-yl)oxy)-2,2-dimethyl-N-propyl-propanamide, N-(1-((4-amino-2,2-dioxo-1H-benzo[c][1,2,6]-thiadiazin-5-yl)oxy)-2-methyl-propan-2-yl)isonicotinamide, or any combination thereof.

In some further embodiments, coolness-modifying compositions disclosed herein comprise one or more other umami or kokumi enhancing compounds. Such umami enhancing compounds include, but are not limited to, naturally derived compounds, such as ericamide, or synthetic compounds, such as any compounds set forth in U.S. Patent Nos. 8,735,081; 8,124,121; and 8,968,708.

In some further embodiments, coolness-modifying compositions disclosed herein comprise one or more cooling enhancing compounds. Such cooling enhancing compounds include, but are not limited to, naturally derived compounds, such as menthol or analogs thereof, or synthetic compounds, such as any compounds set forth in U.S. Patent Nos. 9,394,287 and 10,421,727.

In some further embodiments, coolness-modifying compositions disclosed herein comprise one or more bitterness blocking compounds. Such bitterness blocking compounds include, but are not limited to, naturally derived compounds, such as menthol or analogs thereof, or synthetic compounds, such as any compounds set forth in U.S. Patent Nos. 8,076,491; 8,445,692; and 9,247,759. Examples include N-ethyl-N-(thiophen-2-ylmethyl)-2-(p-tolyloxy)acetamide; N-(1H-pyrazol-3-yl)-N-(thiophen-2-ylmethyl)-2-(p-tolyloxy)acetamide; 2-(4-fluorophenoxy)-N-(1H-pyrazol-3-yl)-N-(thiophen-2-ylmethyl)acetamide; 2-(2-hydroxy-4-methylphenoxy)-N-(1H-pyrazol-3-yl)-N-(thiophen-2-ylmethyl)-acetamide; 2-((2,3-dihydro-1H-inden-5-yl)oxy)-N-(1H-pyrazol-3-yl)-N-(thiophen-2-ylmethyl)-acetamide; 2-((2,3-dihydro-1H-inden-5-yl)oxy)-N-(1H-pyrazol-3-yl)-N-(thiazol-5-ylmethyl)-acetamide; 2-((5-methoxybenzofuran-2-yl)oxy)-N-(1H-pyrazol-3-yl)-N-(thiophen-2-ylmethyl)-acetamide; and any combinations thereof.

In some further embodiments, mouthfeel-modifying compositions disclosed herein comprise one or more mouthfeel modifying compounds. Such mouthfeel modifying compounds include, but are not limited to, tannins, cellulosic materials, bamboo powder, and the like.

In some further embodiments, coolness-modifying compositions disclosed herein comprise one or more flavor masking compounds. Such flavor masking compounds include, but are not limited to, cellulosic materials, materials extracted from fungus, materials extracted from plants, citric acid, carbonic acid (or carbonates), and the like.

The coolness-modifying compositions disclosed herein can, in certain embodiments, comprise cyclosal. When cyclosal is present, it is present at a relative concentration similar to that of the lactones.

The coolness-modifying composition can be in any suitable form. For example, in some embodiments, the composition is in the form of a solid, such as a solid powder. In some such embodiments, the composition also comprises a solid carrier, such as a carbohydrate. In some other embodiments, the coolness-modifying composition is in the form of a liquid solution or suspension. Such a liquid form can be aqueous or non-aqueous, or can also be an emulsion, such as an oil-in-water or water-in-oil emulsion. In some embodiments, the coolness-modifying composition is incorporated into a flavored product, such as a food or beverage product, or an oral care product, such as a toothpaste or mouthwash.

In some embodiments, the coolness-modifying composition comprises one or more flavorings, or any other additives described in H. Mitchell, SWEETENERS AND SUGAR ALTERNATIVES IN FOOD TECHNOLOGY, Blackwell Publishing Ltd, 2006. As used herein, the term "flavorings" includes those flavors known to the skilled person, such as natural and artificial flavors. These flavorings may be chosen from synthetic flavor oils and flavoring aromatics or oils, oleoresins and extracts derived from plants, leaves, flowers, fruits, and so forth, and combinations thereof. Non-limiting representative flavor oils include spearmint oil, cinnamon oil, oil of wintergreen (methyl salicylate), peppermint oil, Japanese mint oil, clove oil, bay oil, anise oil, eucalyptus oil, thyme oil, cedar leaf oil, oil of nutmeg, allspice, oil of sage, mace, oil of bitter almonds, and cassia oil. Also useful flavorings are artificial, natural and synthetic fruit flavors such as vanilla, and citrus oils including lemon, orange, lime, grapefruit, yazu, sudachi, and fruit essences including apple, pear, peach, grape, blueberry, strawberry, raspberry, cherry, plum, pineapple, watermelon, apricot, banana, melon, apricot, ume, cherry, raspberry, blackberry, tropical fruit, mango, mangosteen, pomegranate, papaya and so forth. Other potential flavors include a milk flavor, a butter flavor, a cheese flavor, a cream flavor, and a yogurt flavor; a vanilla flavor; tea or coffee flavors, such as a green tea flavor, a oolong tea flavor, a tea flavor, a cocoa flavor, a chocolate flavor, and a coffee flavor; mint flavors, such as a peppermint flavor, a spearmint flavor, and a Japanese mint flavor; spicy flavors, such as an asafetida flavor, an ajowan flavor, an anise flavor, an angelica flavor, a fennel flavor, an allspice flavor, a cinnamon flavor, a camomile flavor, a mustard flavor, a cardamom flavor, a caraway flavor, a cumin flavor, a clove flavor, a pepper flavor, a coriander flavor, a sassafras flavor, a savory flavor, a Zanthoxyli Fructus flavor, a perilla flavor, a juniper berry flavor, a ginger flavor, a star anise flavor, a horseradish flavor, a thyme flavor, a tarragon flavor, a dill flavor, a capsicum flavor, a nutmeg flavor, a basil flavor, a marjoram flavor, a rosemary flavor, a bayleaf flavor, and a wasabi (Japanese horseradish) flavor; alcoholic flavors, such as a wine flavor, a whisky flavor, a brandy flavor, a rum flavor, a gin flavor, and a liqueur flavor; floral flavors; and vegetable flavors, such as an onion flavor, a garlic flavor, a cabbage flavor, a carrot flavor, a celery flavor, mushroom flavor, and a tomato flavor. These flavoring agents may be used in liquid or solid form and may be used individually or in admixture. Commonly used flavors include mints such as peppermint, menthol, spearmint, artificial vanilla, cinnamon derivatives, and various fruit flavors, whether employed individually or in admixture. Flavors may also provide breath freshening properties, particularly the mint flavors when used in combination with cooling agents.

Flavors may also provide breath freshening properties, particularly the mint flavors when used in combination with cooling agents. These flavorings may be used in liquid or solid form and may be used individually or in admixture. Other useful flavorings include aldehydes and esters such as cinnamyl acetate, cinnamaldehyde, citral diethylacetal, dihydrocarvyl acetate, eugenyl formate, p-methylamisol, and so forth may be used. Generally, any flavoring or food additive such as those described in CHEMICALS USED IN FOOD PROCESSING, publication 1274, pages 63-258, by the National Academy of Sciences, may be used.

Further examples of aldehyde flavorings include but are not limited to acetaldehyde (apple), benzaldehyde (cherry, almond), anisic aldehyde (licorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavors), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2 (berry fruits), tolyl aldehyde (cherry, almond), veratraldehyde (vanilla), 2,6- dimethyl-5-heptenal, i.e., melonal (melon), 2,6-dimethyloctanal (green fruit), and 2- dodecenal (citrus, mandarin), cherry, grape, strawberry shortcake, and mixtures thereof. These listings of flavorings are merely exemplary and are not meant to limit either the term "flavoring" or the scope of the disclosure generally.

In certain embodiments, mint flavorings, including menthol and related compounds, or other breath-freshening compounds, can be combined with cooling agents, such as those set forth in U.S. Patent Nos. 9,394,287 and 9,732,071. Additional examples of cooling agents include 5-methyl-2-(propane-2-yl)cyclohexyl-N-ethyloxamate, N-ethyl-p-menthane carboxamide (WS-3, also referred to as menthane-3-carboxylic acid-N-ethyl amide), N-2,3-trimethyl-2-isopropyl butane amide (WS-23), menthyl lactate (Frescolat.RTM. ML), menthone glycerine acetal (Frescolat.RTM. MGA), mono-menthyl succinate (Physcool.RTM.), mono-menthyl glutarate, O-menthyl-glycerine, menthyl-N,N-dimethyl succinamate, N-(4-cyano methyl phenyl)-p-menthane carboxamide, N-(2-(pyridin-2-yl)ethyl)-3-p-menthane carboxamide, menthol and menthol derivatives (e.g. L-menthol, D-menthol, racemic menthol, isomenthol, neoisomenthol, neomenthol) menthyl ether (e.g. (I-menthoxy)-1,2-propanediol, (I-menthoxy)-2-methyl-1,2-propanediol, 1-menthyl-methyl ether), menthyl ester (e.g. menthyl formiate, menthyl acetate, menthyl isobutyrate, menthyl lactates, L-menthyl-L-lactate, L-menthyl-D-lactate, menthyl-(2-methoxy)acetate, menthyl-(2-methoxy ethoxy)acetate, menthyl pyroglutamate), N-(4-cyano methyl phenyl)-p-menthane carboxamide, N-(2-(pyridin-2-yl)ethyl)-3-p-menthane carboxamides, menthyl carbonates (e.g. menthyl propylene glycol carbonate, menthyl ethylene glycol carbonate, menthyl glycerine carbonate or mixtures thereof), menthane carboxylic acid amide (e.g. menthane carboxylic acid-N-ethylamid [WS3], N-alpha.-(menthane-carbonyl)glycine ethyl ester [WS5], menthane carboxylic acid-N-(4-cyanophenyl)amide, menthane carboxylic acid-N-(alkoxyalkyl)amide), menthone and menthone derivatives (e.g. L-menthone glycerine ketal), 2,3-dimethyl-2-(2-propyl)-butyric acid derivatives (e.g. 2,3-dimethyl-2-(2-propyl)-butyric acid-N-methyl amide [WS23]), isopulegol or its esters (1-(-)-isopulegol, 1-(-)-isopulegol acetate), menthane derivatives (e.g. p-menthane-3,8-diol), N-(4-cyano methyl phenyl)-p-menthane carboxamides, N-(2-(pyridin-2-yl)ethyl)-3-p-menthane carboxamides, cubebol or synthetic or natural mixtures containing cubebol, pyrrolidone derivates of cycloalkyl dione derivatives (e.g. 3-methyl-2(1-pyrrolidinyl)-2-cyclopentene-1-one) or tetrahydropyrimidine-2-ones (e.g. Icilin or related compounds such as those described in WO 2004/026840), N-(4-cyano methyl phenyl)-p-menthane carboxamide, N-(2-(pyridin-2-yl)ethyl)-3-p-menthane carboxamides, menthyl ether (e.g. (I-menthoxy)-1,2-propanediol, (I-menthoxy)-2-methyl-1,2-propanediol), more polar menthyl esters (e.g. menthyl lactates, L-menthyl-L-lactate, L-menthyl-D-lactate, menthyl-(2-methoxy)acetate, menthyl-(2-methoxy ethoxy)acetate, menthyl pyroglutamate), menthyl carbonates (e.g. menthyl propylene glycol carbonate, menthyl ethylene glycol carbonate, menthyl glycerine carbonate), the semi-esters of menthols with a dicarboxylic acid or the derivatives thereof (e.g. mono-menthyl succinate, mono-menthyl glutarate, mono-menthyl malonate, O-menthyl succinic acid ester-N,N-(dimethyl)amide, O-menthyl succinic acid ester amide), 3,4-methylendioxycinnamic acid-N-cyclohexyl-N-2-pyridylamide, isopropyl-(5-methoxy-2-pyridin-2-yl-pyrimidin-4-yl)-amine, 3,4,6,7,11b,12-hexahydro-3,3-dimethyl-spiro[13H-dibenzo[a,f]quinolizine-1--3,2'-[1,3]dithiolan]-1(2H)-one, 5,6,10b,11-tetrahydro-3-methyl-spiro[12H-benzo[a]furo[3,4-flquinolizine-1- -2,2'-[1,3]dithiolan]-1(3H)-one. Most preferred as cooling compounds are compounds selected from the group consisting of 5-methyl-2-(propane-2-yl)cyclohexyl-N-ethyloxamate, N-ethyl-p-menthane carboxamide (WS-3, also referred to as menthane-3-carboxylic acid-N-ethyl amide), menthyl lactate (Frescolat.RTM. ML), menthone glycerine acetal (Frescolat.RTM. MGA), N-(4-cyano methyl phenyl)-p-menthane carboxamide and (I-menthoxy)-1,2-propanediol.

### Coolness-Modifying Compositions Suitable for Body Care Products

In some embodiments of any of the foregoing embodiments, the coolness-modifying compositions comprise any compounds, materials, or combinations thereof that are commonly included in body care products, such as products suitable for application to human hair or skin.

In some embodiments, the composition comprises one or more fragrances. In some embodiments, at least a portion of the fragrances can be encapsulated. Suitable fragrances include complex mixtures of fragrance compounds, chosen to provide any desired odor. In the context of the present disclosure the term "fragrance" is used synonymously with "perfume." Fragrance compounds typically used in the field of perfumery and suitable for the purposes of the present disclosure are described more fully in S. Arctander, PERFUME FLAVORS AND CHEMICALS, Vols. I and II (1969), and in THE MERCK INDEX, 8th edition, Merck & Co., Inc. Rahway, N.J. The term "fragrance compound" encompasses naturally occurring as well as synthetic materials known for use in perfumes, as well as animal oils. A fragrance compound can also be any natural oil or extract, or chemical compound used in a fragrance composition. Natural oils and extracts are described in Guenther, THE ESSENTIAL OILS 1949, and can include extracts, pressings, collection of exudates, and distillates from any part of suitable plants: roots, rhizomes, bulbs, corms, stem, bark, heartwood, leaves, flowers, seeds and fruit. Examples of such extracts and distillates include citrus fruit oils such as orange, mandarin, grapefruit, lime or lemon oils, tree oils such as pine, or cedarwood, herb oils such as peppermint, thyme, lavender, basil, rosemary, clove or flower extracts such as rose, jasmine, muguet, or geranium oil.

In some embodiments, the body care compositions can include one or more ingredients that provide an active benefit to hair or skin care, such as compounds having antioxidant activity. For example, in some embodiments, the body care product comprises ascorbic acid, also known as Vitamin C, which is an essential nutrient with antioxidant activity. In some embodiments, the body care composition comprises vitamin E, which refers to eight structurally-related antioxidant compounds that exhibit radical-scavenging activity. The compounds include four tocopherol compounds and four tocotrienol compounds. In some embodiments, the body care composition comprises resveratrol, which is is an antioxidant compound that occurs naturally in grape skin. In some embodiments, the body care composition comprises certain oligopeptides, such as acetyl hexapeptide-8 (also referred to as acetyl hexapeptide-3), which is a synthetic peptide that relaxes wrinkles. A variety of other such compounds can be included too, such as retinol, niacinamide, coenzyme Q10, and various polyphenols, such as flavonoids like catechins. These active ingredients can be used in amounts typical of their use in other body care products.

The body care compositions disclosed herein can include or be incorporated into any suitable type of vehicle and carrier. In general, the vehicle or carrier will be a dermatologically acceptable vehicle or carrier. Non-limiting examples of vehicles or carriers include water, glycerin, alcohol, oil, silicon-containing compounds, silicone compounds, and waxes. Variations and other appropriate vehicles will be apparent to the skilled artisan and are appropriate for use in the body care compositions of the present disclosure. In certain embodiments, the concentrations and combinations of the compounds, ingredients, and agents can be selected in such a way that the combinations are chemically compatible and do not form complexes which precipitate from the finished body care product.

The body care compositions disclosed herein can be structured or formulated into a variety of different forms. Non-limiting examples include emulsions (for example, water-in-oil, water-in-oil-in-water, oil-in-water, silicone-in-water, water-in-silicone, oil-in-water-in-oil, oil-in-water-in-silicone emulsions, and the like), creams, lotions, solutions (for example, aqueous or hydro-alcoholic), anhydrous bases (such as lipsticks and powders), gels, masks, scrubs, body butters, peels, and ointments. Variations and other structures will be apparent to the skilled artisan and are appropriate for use in the body care compositions of the present disclosure.

A wide variety of other ingredients can be used, such as ingredients typically used in cosmetics, skin care products, or hair care products. CTFA INTERNATIONAL COSMETIC INGREDIENT DICTIONARY AND HANDBOOK (2004 and 2008) describe a wide variety of non-limiting ingredients that can be used in the presently disclosed compositions. Examples of these ingredient classes include: fragrance agents (artificial and natural; e.g., gluconic acid, phenoxyethanol, and triethanolamine), dyes and color ingredients (e.g., Blue 1, Blue 1 Lake, Red 40, titanium dioxide, D&C blue no. 4, D&C green no. 5, D&C orange no. 4, D&C red no. 17, D&C red no. 33, D&C violet no. 2, D&C yellow no. 10, and D&C yellow no. 11), flavoring agents or aroma agents (e.g., Stevia rebaudiana (sweetleaf) extract, and menthol), adsorbents, lubricants, solvents, moisturizers (including, e.g., emollients, humectants, film formers, occlusive agents, and agents that affect the natural moisturization mechanisms of the skin), water-repellants, UV absorbers (physical and chemical absorbers such as paraaminobenzoic acid ("PABA") and corresponding PABA derivatives, titanium dioxide, zinc oxide, etc.), essential oils, vitamins (e.g., B, D, E, and K), trace metals (e.g., zinc, calcium and selenium), anti-irritants (e.g., steroids and non-steroidal anti-inflammatories), botanical extracts (e.g., Aloe vera, chamomile, cucumber extract, Ginkgo biloba, ginseng, and rosemary), anti-microbial agents, antioxidants (e.g., BHT and tocopherol), chelating agents (e.g., disodium EDTA and tetrasodium EDTA), preservatives (e.g., methylparaben and propylparaben), pH adjusters (e.g., sodium hydroxide and citric acid), absorbents (e.g., aluminum starch octenylsuccinate, kaolin, corn starch, oat starch, cyclodextrin, talc, and zeolite), skin bleaching and lightening agents (e.g., hydroquinone and niacinamide lactate), humectants (e.g., sorbitol, urea, methyl gluceth-20, saccharide isomerate, and mannitol), exfoliants, waterproofing agents (e.g., magnesium/aluminum hydroxide stearate), skin conditioning agents (e.g., aloe extracts, allantoin, bisabolol, ceramides, dimethicone, hyaluronic acid, biosaccharide gum-1, ethylhexylglycerin, pentylene glycol, hydrogenated polydecene, octyldodecyl oleate, and dipotassium glycyrrhizate). Further non-limiting examples of some of these ingredients are provided in the following subsections.

In some embodiments, the body care compositions comprise one or more UV absorption or reflecting agents. Suitable UV absorption or reflecting agents include chemical and physical sunblocks. Non-limiting examples of chemical sunblocks include paraaminobenzoic acid (PABA), PABA esters (glyceryl PABA, amyldimethyl PABA and octyldimethyl PABA), butyl PABA, ethyl PABA, ethyl dihydroxypropyl PABA, benzophenones (oxybenzone, sulisobenzone, benzophenone, and benzophenone-1 through 12), cinnamates (octyl methoxycinnamate (octinoxate), isoamyl p-methoxycinnamate, octylmethoxy cinnamate, cinoxate, diisopropyl methyl cinnamate, DEA-methoxycinnamate, ethyl diisopropylcinnamate, glyceryl octanoate dimethoxycinnamate and ethyl methoxycinnamate), cinnamate esters, salicylates (homomethyl salicylate, benzyl salicylate, glycol salicylate, isopropylbenzyl salicylate, etc.), anthranilates, ethyl urocanate, homosalate, octisalate, dibenzoylmethane derivatives (e.g., avobenzone), octocrylene, octyl triazone, digalloyl trioleate, glyceryl aminobenzoate, lawsone with dihydroxyacetone, ethylhexyl triazone, dioctyl butamido triazone, benzylidene malonate polysiloxane, terephthalylidene dicamphor sulfonic acid, disodium phenyl dibenzimidazole tetrasulfonate, diethylamino hydroxybenzoyl hexyl benzoate, bis diethylamino hydroxybenzoyl benzoate, bis benzoxazoylphenyl ethylhexylimino triazine, drometrizole trisiloxane, methylene bisbenzotriazolyl tetramethylbutylphenol, and bis-ethylhexyloxyphenol methoxyphenyltriazine, 4-methylbenzylidene camphor, and isopentyl 4-methoxycinnamate. Non-limiting examples of physical sunblocks include, kaolin, talc, petrolatum, and metal oxides (e.g., titanium dioxide and zinc oxide).

In some embodiments, the body care composition comprises one or more moisturizing agents. Non-limiting examples of moisturizing agents that can be used include amino acids, chondroitin sulfate, diglycerin, erythritol, fructose, glucose, glycerin, glycerol polymers, glycol, 1,2,6-hexanetriol, honey, hyaluronic acid, hydrogenated honey, hydrogenated starch hydrolysate, inositol, lactitol, maltitol, maltose, mannitol, natural moisturizing factor, PEG-15 butanediol, polyglyceryl sorbitol, salts of pyrrolidone carboxylic acid, potassium PCA, propylene glycol, saccharide isomerate, sodium glucuronate, sodium PCA, sorbitol, sucrose, trehalose, urea, and xylitol. Other non-limiting examples include acetylated lanolin, acetylated lanolin alcohol, alanine, algae extract, Aloe barbadensis, Aloe barbadensis extract, Aloe barbadensis gel, Althea officinalis extract, apricot (Prunus armeniaca) kernel oil, arginine, arginine aspartate, Arnica montana extract, aspartic acid, avocado (Persea gratissima) oil, barrier sphingolipids, butyl alcohol, beeswax, behenyl alcohol, beta-sitosterol, birch (Betula alba) bark extract, borage (Borago officinalis) extract, butcherbroom (Ruscus aculeatus) extract, butylene glycol, Calendula officinalis extract, Calendula officinalis oil, candelilla (Euphorbia cerifera) wax, canola oil, caprylic/capric triglyceride, cardamom (Elettaria cardamomum) oil, carnauba (Copemicia cerifera) wax, carrot (Daucus carota sativa) oil, castor (Ricinus communis) oil, ceramides, ceresin, ceteareth-5, ceteareth-12, ceteareth-20, cetearyl octanoate, ceteth-20, ceteth-24, cetyl acetate, cetyl octanoate, cetyl palmitate, chamomile (Anthemis nobilis) oil, cholesterol, cholesterol esters, cholesteryl hydroxystearate, citric acid, clary (Salvia sclarea) oil, cocoa (Theobroma cacao) butter, cococaprylate/caprate, coconut (Cocos nucifera) oil, collagen, collagen amino acids, corn (Zea mays) oil, fatty acids, decyl oleate, dimethicone copolyol, dimethiconol, dioctyl adipate, dioctyl succinate, dipentaerythrityl hexacaprylate/hexacaprate, DNA, erythritol, ethoxydiglycol, ethyl linoleate, Eucalyptus globulus oil, evening primrose (Oenothera biennis) oil, fatty acids, Geranium maculatum oil, glucosamine, glucose glutamate, glutamic acid, glycereth-26, glycerin, glycerol, glyceryl distearate, glyceryl hydroxystearate, glyceryl laurate, glyceryl linoleate, glyceryl myristate, glyceryl oleate, glyceryl stearate, glyceryl stearate SE, glycine, glycol stearate, glycol stearate SE, glycosaminoglycans, grape (Vitis vinifera) seed oil, hazel (Corylus americana) nut oil, hazel (Corylus avellana) nut oil, hexylene glycol, hyaluronic acid, hybrid safflower (Carthamus tinctorius) oil, hydrogenated castor oil, hydrogenated coco-glycerides, hydrogenated coconut oil, hydrogenated lanolin, hydrogenated lecithin, hydrogenated palm glyceride, hydrogenated palm kernel oil, hydrogenated soybean oil, hydrogenated tallow glyceride, hydrogenated vegetable oil, hydrolyzed collagen, hydrolyzed elastin, hydrolyzed glycosaminoglycans, hydrolyzed keratin, hydrolyzed soy protein, hydroxylated lanolin, hydroxyproline, isocetyl stearate, isocetyl stearoyl stearate, isodecyl oleate, isopropyl isostearate, isopropyl lanolate, isopropyl myristate, isopropyl palmitate, isopropyl stearate, isostearamide DEA, isostearic acid, isostearyl lactate, isostearyl neopentanoate, jasmine (Jasminum officinale) oil, jojoba (Buxus chinensis) oil, kelp, kukui (Aleurites moluccana) nut oil, lactamide MEA, laneth-16, laneth-10 acetate, lanolin, lanolin acid, lanolin alcohol, lanolin oil, lanolin wax, lavender (Lavandula angustifolia) oil, lecithin, lemon (Citrus medica limonum) oil, linoleic acid, linolenic acid, Macadamia ternifolia nut oil, maltitol, matricaria (Chamomilla recutita) oil, methyl glucose sesquistearate, methylsilanol PCA, mineral oil, mink oil, mortierella oil, myristyl lactate, myristyl myristate, myristyl propionate, neopentyl glycol dicaprylate/dicaprate, octyldodecanol, octyldodecyl myristate, octyldodecyl stearoyl stearate, octyl hydroxystearate, octyl palmitate, octyl salicylate, octyl stearate, oleic acid, olive (Olea europaea) oil, orange (Citrus aurantium dulcis) oil, palm (Elaeis guineensis) oil, palmitic acid, pantethine, panthenol, panthenyl ethyl ether, paraffin, PCA, peach (Prunus persica) kernel oil, peanut (Arachis hypogaea) oil, PEG-8 C12-18 ester, PEG-15 cocamine, PEG-150 distearate, PEG-60 glyceryl isostearate, PEG-5 glyceryl stearate, PEG-30 glyceryl stearate, PEG-7 hydrogenated castor oil, PEG-40 hydrogenated castor oil, PEG-60 hydrogenated castor oil, PEG-20 methyl glucose sesquistearate, PEG-40 sorbitan peroleate, PEG-5 soy sterol, PEG-10 soy sterol, PEG-2 stearate, PEG-8 stearate, PEG-20 stearate, PEG-32 stearate, PEG-40 stearate, PEG-50 stearate, PEG-100 stearate, PEG-150 stearate, pentadecalactone, peppermint (Mentha piperita) oil, petrolatum, phospholipids, plankton extract, polyamino sugar condensate, polyglyceryl-3 diisostearate, polyquaternium-24, polysorbate 20, polysorbate 40, polysorbate 60, polysorbate 80, polysorbate 85, potassium myristate, potassium palmitate, propylene glycol, propylene glycol dicaprylate/dicaprate, propylene glycol dioctanoate, propylene glycol dipelargonate, propylene glycol laurate, propylene glycol stearate, propylene glycol stearate SE, PVP, pyridoxine dipalmitate, retinol, retinyl palmitate, rice (Oryza sativa) bran oil, RNA, rosemary (Rosmarinus officinalis) oil, rose oil, safflower (Carthamus tinctorius) oil, sage (Salvia officinalis) oil, sandalwood (Santalum album) oil, serine, serum protein, sesame (Sesamum indicum) oil, shea butter (Butyrospermum parkii), silk powder, sodium chondroitin sulfate, sodium hyaluronate, sodium lactate, sodium palmitate, sodium PCA, sodium polyglutamate, soluble collagen, sorbitan laurate, sorbitan oleate, sorbitan palmitate, sorbitan sesquioleate, sorbitan stearate, sorbitol, soybean (Glycine soja) oil, sphingolipids, squalane, squalene, stearamide MEA-stearate, stearic acid, stearoxy dimethicone, stearoxytrimethylsilane, stearyl alcohol, stearyl glycyrrhetinate, stearyl heptanoate, stearyl stearate, sunflower (Helianthus annuus) seed oil, sweet almond (Prunus amygdalus dulcis) oil, synthetic beeswax, tocopheryl acetate, tocopheryl linoleate, tribehenin, tridecyl neopentanoate, tridecyl stearate, triethanolamine, tristearin, urea, vegetable oil, water, waxes, wheat (Triticum vulgare) germ oil, and ylang (Cananga odorata) oil.

In some embodiments, the body care compositions disclosed herein comprise one or more antioxidants, for example, in addition to or in combination with those disclosed above. For example, in some embodiments, one or more of these additional antioxidants can be used in combination with ascorbic acid, vitamin E, or combinations thereof. Non-limiting examples of antioxidants that can be used include acetyl cysteine, ascorbic acid derivatives like ascorbic acid polypeptide, ascorbyl dipalmitate, ascorbyl methylsilanol pectinate, ascorbyl palmitate, ascorbyl stearate, BHA, BHT, t-butyl hydroquinone, cysteine, cysteine HCl, diamylhydroquinone, di-t-butylhydroquinone, dicetyl thiodipropionate, dioleyl tocopheryl methylsilanol, disodium ascorbyl sulfate, distearyl thiodipropionate, ditridecyl thiodipropionate, dodecyl gallate, erythorbic acid, esters of ascorbic acid, ethyl ferulate, ferulic acid, gallic acid esters, hydroquinone, isooctyl thioglycolate, kojic acid, magnesium ascorbate, magnesium ascorbyl phosphate, methylsilanol ascorbate, natural botanical antioxidants such as green tea or grape seed extracts, nordihydroguaiaretic acid, octyl gallate, phenylthioglycolic acid, potassium ascorbyl tocopheryl phosphate, potassium sulfite, propyl gallate, quinones, rosmarinic acid, sodium ascorbate, sodium bisulfite, sodium erythorbate, sodium metabisulfite, sodium sulfite, superoxide dismutase, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocophersolan, tocopheryl acetate, tocopheryl linoleate, tocopheryl nicotinate, tocopheryl succinate, and tris(nonylphenyl)phosphite.

In some embodiments, the body care compositions comprise one or more structuring agents. Structuring agents, in certain embodiments, assist in providing rheological characteristics to the composition that contribute to stability. In some embodiments, structuring agents can also function as an emulsifier or surfactant. Non-limiting examples of structuring agents include stearic acid, palmitic acid, stearyl alcohol, cetyl alcohol, behenyl alcohol, stearic acid, palmitic acid, the polyethylene glycol ether of stearyl alcohol having an average of about 1 to about 21 ethylene oxide units, the polyethylene glycol ether of cetyl alcohol having an average of about 1 to about 5 ethylene oxide units, and mixtures thereof.

In some embodiments, the body care compositions comprise one or more emulsifiers. Emulsifiers can reduce the interfacial tension between phases and improve the formulation and stability of an emulsion. The emulsifiers can be nonionic, cationic, anionic, and zwitterionic emulsifiers. Non-limiting examples include esters of glycerin, esters of propylene glycol, fatty acid esters of polyethylene glycol, fatty acid esters of polypropylene glycol, esters of sorbitol, esters of sorbitan anhydrides, carboxylic acid copolymers, esters and ethers of glucose, ethoxylated ethers, ethoxylated alcohols, alkyl phosphates, polyoxyethylene fatty ether phosphates, fatty acid amides, acyl lactylates, soaps, TEA stearate, DEA oleth-3 phosphate, polyethylene glycol 20 sorbitan monolaurate (polysorbate 20), polyethylene glycol 5 soya sterol, steareth-2, steareth-20, steareth-21, ceteareth-20, cetearyl glucoside, cetearyl alcohol, C₁₂₋₁₃ pareth-3, PPG-2 methyl glucose ether distearate, PPG-5-ceteth-20, bis-PEG/PPG-20/20 dimethicone, ceteth-10, polysorbate 80, cetyl phosphate, potassium cetyl phosphate, diethanolamine cetyl phosphate, polysorbate 60, glyceryl stearate, PEG-100 stearate, arachidyl alcohol, arachidyl glucoside, and mixtures thereof.

In some embodiments, the body care compositions comprise one or more siliconecontaining compounds. Non-limiting examples of silicone containing compounds include any member of a family of polymeric products whose molecular backbone is made up of alternating silicon and oxygen atoms with side groups attached to the silicon atoms. By varying the --Si--O-- chain lengths, side groups, and crosslinking, silicones can be synthesized into a wide variety of materials. They can vary in consistency from liquid to gel to solids. The silicone containing compounds suitable for use in the body care compositions disclosed herein include, but are not limited to, those generally known to a person of ordinary skill in the art. Non-limiting examples include silicone oils (e.g., volatile and non-volatile oils), gels, and solids. In certain embodiments, the silicon-containing compounds include silicone oils such as a polyorganosiloxane. Non-limiting examples of polyorganosiloxanes include dimethicone, cyclomethicone, polysilicone-11, phenyl trimethicone, trimethylsilylamodimethicone, stearoxytrimethylsilane, or mixtures of these and other organosiloxane materials in any given ratio in order to achieve the desired consistency and application characteristics depending upon the intended application (e.g., to a particular area such as the skin, hair, or eyes). A "volatile silicone oil" includes a silicone oil having a low heat of vaporization, such as less than about 50 cal/g of silicone oil. Non-limiting examples of volatile silicone oils include cyclomethicones; low viscosity dimethicones, such as dimethicones having a viscosity of about 50 cst or less. Other non-limiting volatile silicone oils that can be used in the context of the present invention include those available from manufacturers like Dow Corning, General Electric, and SWS Silicones.

In some embodiments, the body care composition comprises one or more exfoliating agents. Exfoliating agents include ingredients that remove dead skin cells on the skin's outer surface. These agents may act through mechanical, chemical, or other means. Non-limiting examples of mechanical exfoliating agents include abrasives such as pumice, silica, cloth, paper, shells, beads, solid crystals, solid polymers, etc. Non-limiting examples of chemical exfoliating agents include acids and enzyme exfoliants. Acids that can be used as exfoliating agents include, but are not limited to, glycolic acid, lactic acid, citric acid, alpha hydroxy acids, beta hydroxy acids, etc. Other exfoliating agents known to those of skill in the art are also contemplated as being useful within the context of the body care compositions of the present disclosure.

In some embodiments, the body care compositions include one or more essential oils. Essential oils include oils derived from herbs, flowers, trees, and other plants. Such oils are typically present as tiny droplets between the plant's cells, and can be extracted by several method known to those of skill in the art (for example, steam distilled, enfleurage (for example, extraction by using fat), maceration, solvent extraction, or mechanical pressing). When these types of oils are exposed to air they tend to evaporate (for example, a volatile oil). Essential oils are generally insoluble in water, but are soluble in alcohol, ether, fixed oils (vegetal), and other organic solvents. Essential oils are typically named by the plant from which the oil is found. Non-limiting examples of essential oils that can be used in the context of the body care compositions of the present disclosure include sesame oil, macadamia nut oil, tea tree oil, evening primrose oil, Spanish sage oil, Spanish rosemary oil, coriander oil, thyme oil, pimento berries oil, rose oil, anise oil, balsam oil, bergamot oil, rosewood oil, cedar oil, chamomile oil, sage oil, clary sage oil, clove oil, cypress oil, eucalyptus oil, fennel oil, sea fennel oil, frankincense oil, geranium oil, ginger oil, grapefruit oil, jasmine oil, juniper oil, lavender oil, lemon oil, lemongrass oil, lime oil, mandarin oil, marjoram oil, myrrh oil, neroli oil, orange oil, patchouli oil, pepper oil, black pepper oil, petitgrain oil, pine oil, rose otto oil, rosemary oil, sandalwood oil, spearmint oil, spikenard oil, vetiver oil, wintergreen oil, and ylang. Other essential oils known to those of skill in the art are also contemplated as being useful within the context of the present invention.

In some embodiments, the body care compositions comprise one or more thickening agents. Thickening agents, including thickener or gelling agents, include substances that increase the viscosity of a composition. Thickeners includes those that increase the viscosity of a composition without substantially modifying the efficacy of the active ingredient within the composition. Thickeners can also increase the stability of the compositions. Commonly used thickeners include hydrogenated polyisobutene, trihydroxystearin, ammonium acryloyldimethyltaurate/vp copolymer, or combinations thereof. Other non-limiting examples of additional thickening agents that can be used include carboxylic acid polymers, crosslinked polyacrylate polymers, polyacrylamide polymers, polysaccharides, and gums. Examples of carboxylic acid polymers include crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol. Examples of commercially available carboxylic acid polymers include carbomers, which are homopolymers of acrylic acid crosslinked with allyl ethers of sucrose or pentaerythritol. Non-limiting examples of crosslinked polyacrylate polymers include cationic and nonionic polymers. Non-limiting examples are described in U.S. Pat. Nos. 5,100,660; 4,849,484; 4,835,206; 4,628,078; 4,599,379. Non-limiting examples of polyacrylamide polymers (including nonionic polyacrylamide polymers including substituted branched or unbranched polymers) include polyacrylamide, isoparaffin and laureth-7, multi-block copolymers of acrylamides and substituted acrylamides with acrylic acids and substituted acrylic acids. Non-limiting examples of polysaccharides include cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Another example is an alkyl substituted cellulose where the hydroxy groups of the cellulose polymer is hydroxyalkylated (such as hydroxy ethylated or hydroxypropylated) to form a hydroxyalkylated cellulose which is then further modified with a C10-C30 straight chain or branched chain alkyl group through an ether linkage. Typically these polymers are ethers of C₁₀-C₃₀ straight or branched chain alcohols with hydroxyalkylcelluloses. Other useful polysaccharides include scleroglucans comprising a linear chain of (1-3) linked glucose units with a (1-6) linked glucose every three units. Non-limiting examples of gums that can be used include acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluronic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboxymethyl dextran, sodium carrageenan, tragacanth gum, xanthan gum, and mixtures thereof.

In some embodiments, the body care compositions comprise one or more preservatives. Non-limiting examples of preservatives that can be used in the context of the present invention include quaternary ammonium preservatives such as polyquaternium-1 and benzalkonium halides (for example, benzalkonium chloride and benzalkonium bromide), parabens (for example, methylparabens and propylparabens), phenoxyethanol, benzyl alcohol, chlorobutanol, phenol, sorbic acid, thimerosal, or combinations thereof.

In some embodiments, the body care compositions comprise one or more emollients. Non-limiting examples of suitable emollients include the following: (a) silicone oils and modifications thereof such as linear and cyclic polydimethylsiloxanes; amino, alkyl, alkylaryl, and aryl silicone oils; (b) fats and oils including natural fats and oils such as jojoba, soybean, sunflower, rice bran, avocado, almond, olive, sesame, persic, castor, coconut, mink oils; cacao fat; beef tallow, lard; hardened oils obtained by hydrogenating the aforementioned oils; and synthetic mono, di and triglycerides such as myristic acid glyceride and 2-ethylhexanoic acid glyceride; (c) waxes such as carnauba, spermaceti, beeswax, lanolin, and derivatives thereof; (d) hydrophobic plant extracts; (e) hydrocarbons such as liquid paraffins, vaseline, microcrystalline wax, ceresin, squalene, pristan and mineral oil; (f) higher fatty acids such as lauric, myristic, palmitic, stearic, behenic, oleic, linoleic, linolenic, lanolic, isostearic, arachidonic and poly unsaturated fatty acids (PUFA); (g) higher alcohols such as lauryl, cetyl, stearyl, oleyl, behenyl, cholesterol and 2-hexydecanol alcohol; (h) esters such as cetyl octanoate, myristyl lactate, cetyl lactate, isopropyl myristate, myristyl myristate, isopropyl palmitate, isopropyl adipate, butyl stearate, decyl oleate, cholesterol isostearate, glycerol monostearate, glycerol distearate, glycerol tristearate, alkyl lactate, alkyl citrate and alkyl tartrate; (i) essential oils and extracts thereof such as mentha, jasmine, camphor, white cedar, bitter orange peel, ryu, turpentine, cinnamon, bergamot, citrus unshiu, calamus, pine, lavender, bay, clove, hiba, eucalyptus, lemon, starflower, thyme, peppermint, rose, sage, sesame, ginger, basil, juniper, lemon grass, rosemary, rosewood, avocado, grape, grapeseed, myrrh, cucumber, watercress, calendula, elder flower, geranium, linden blossom, amaranth, seaweed, ginko, ginseng, carrot, guarana, tea tree, jojoba, comfrey, oatmeal, cocoa, neroli, vanilla, green tea, penny royal, aloe vera, menthol, cineole, eugenol, citral, citronelle, borneol, linalool, geraniol, evening primrose, camphor, thymol, spirantol, penene, limonene and terpenoid oils; (j) lipids such as cholesterol, ceramides, sucrose esters and pseudoceramides; (k) vitamins, minerals, and skin nutrients such as vitamins A, E, and K; vitamin alkyl esters, including vitamin C alkyl esters; magnesium, calcium, and milk; (l) sunscreens such as octyl methoxyl cinnamate and butyl methoxy benzoylmethane; (l) phospholipids; (m) polyhydric alcohols such as glycerine and propylene glycol; and polyols such as polyethylene glycols; (n) antiaging compounds such as alpha hydroxy acids, beta hydroxy acids; and (o) mixtures of any of the foregoing components, and the like.

In some embodiments, the body care compositions comprise one or more tackifiers. Non-limiting examples of suitable tackifiers, include aliphatic hydrocarbon resins, aromatic modified aliphatic hydrocarbon resins, hydrogenated polycyclopentadiene resins, polycyclopentadiene resins, gum rosins, gum rosin esters, wood rosins, wood rosin esters, tall oil rosins, tall oil rosin esters, polyterpenes, aromatic modified polyterpenes, terpene phenolics, aromatic modified hydrogenated polycyclopentadiene resins, hydrogenated aliphatic resin, hydrogenated aliphatic aromatic resins, hydrogenated terpenes and modified terpenes, hydrogenated rosin acids, hydrogenated rosin esters, polyisoprene, partially or fully hydrogenated polyisoprene, polybutenediene, partially or fully hydrogenated polybutenediene, and the like. The tackifier may be fully or partially hydrogenated. The tackifier may also be non-polar, non-polar meaning that the tackifier is substantially free of monomers having polar groups. In some embodiments, the polar groups are not present. In some embodiments, the polar groups are present in an amount of up to about 5% by weight, preferably up to about 2% by weight, and more preferably up to about 0.5% by weight.

In some embodiments, the body care compositions comprise one or more colorants, such as pigments or dyestuffs. Examples of suitable pigments include, but are not limited to, inorganic pigments, organic pigments, lakes, pearlescent pigments, iridescent or optically variable pigments, and mixtures thereof. The term "pigment" is understood to mean inorganic or organic, white or colored particles. Said pigments may optionally be surfacetreated within the scope of the present invention but are not limited to treatments such as silicones, perfluorinated compounds, lecithin, and amino acids.

In some embodiments, the body care compositions comprise one or more surfactants. Surfactants useful as the surfactant components in the compositions disclosed herein include nonionic, anionic, cationic, and amphoteric (zwitterionic) surfactants and may be used in combination with each other.

In some embodiments, the body care compositions comprise one of more pH adjustors. The pH adjustors, include inorganic and organic acids and bases and in particular aqueous ammonia, citric acid, phosphoric acid, acetic acid, sodium hydroxide, lactic acid, levulinic acid, glycolic acid, tartaric acid, malic acid, pyrrolidonecarboxylic acid (PCA), succinic acid, citric acid, glutamic acid, 2-amino-2-methyl-1-propanol (AMP), and triethanolamine (TEA).

In some embodiments, the body care compositions comprise one or more reducing agents. Suitable reducing agents include, but are not limited to, thiourea, salts (such as sodium salts) of thiosulfate, sulfite, bisulfite, metabisulfite, borohydride, and hypophosphite, ascorbic acid and salts, esters, and derivatives thereof (for example, ascorbyl palmitate and ascorbyl polypeptide), and tocopherols and salts, esters, and derivatives thereof (for example, tocopherol acetate). Other well-known reducing agents can also be used, such as those set forth in the INCI Handbook.

In some embodiments, the body care compositions comprise one or more fragrances. Non-limiting examples of suitable fragrances include natural oils or naturally derived materials, and synthetic fragrances such as hydrocarbons, alcohols, aldehydes, ketones, esters, lactones, ethers, nitriles, and polyfunctionals. Non-limiting examples of natural oils include the following: basil (Ocimum basilicum) oil, bay (Pimento acris) oil, bee balm (Monarda didyma) oil, bergamot (Citrus aurantium bergamia) oil, cardamom (Elettaria cardamomum) oil, cedarwood (Cedrus atlantica) oil, chamomile (Anthemis nobilis) oil, cinnamon (Cinnamomum cassia) oil, citronella (Cymbopogon nardus) oil, clary (Salvia sclarea) oil, clove (Eugenia caryophyllus) oil, cloveleaf (Eufenia caryophyllus) oil, Cyperus esculentus oil, cypress (Cupressus sempervirens) oil, Eucalyptus citriodora oil, geranium maculatum oil, ginger (Zingiber officinale) oil, grapefruit (Citrus grandis) oil, hazel (Corylus avellana) nut oil, jasmine (Jasminum officinale) oil, Juniperus communis oil, Juniperus oxycedrus tar, Juniperus virginiana oil, kiwi (Actinidia chinensis) water, lavandin (Lavandula hybrida) oil, lavender (Lavandula angustifolia) oil, lavender (Lavandula angustifolia) water, lemon (Citrus medica limonum) oil, lemongrass (Cymbopogon schoenanthus) oil, lime (Citrus aurantifolia) oil, linden (Tilia cordata) oil, linden (Tilia cordata) water, mandarin orange (Citrus nobilis) oil, nutmeg (Myristica fragrans) oil, orange (Citrus aurantium dulcis) flower oil, orange (Citrus aurantium dulcis) oil, orange (Citrus aurantium dulcis) water, patchouli (Pogostemon cablin) oil, peppermint (Menthe piperita) oil, peppermint (Menthe peperita) water, rosemary (Rosmarinus officinalis) oil, rose oil, rose (Rosa damascena) extract, rose (Rosa multiflora) extract, rosewood (Aniba rosaeodora) extract, sage (Salvia officinalis) oil, sandalwood (Santalum album) oil, spearmint (Menthe viridis) oil, tea tree (Melaleuca alternifolia) oil, and ylang (Cananga odorata) oil. Some non-limiting examples of synthetic hydrocarbon fragrances include caryophyllene, .beta.-farnesene, limonene, .alpha.-pinene, and, .beta.-pinene. Some non-limiting examples of synthetic alcohol fragrances include bacdanol, citronellol, linalool, phenethyl alcohol, and .alpha.-terpineol (R.dbd.H). Some non-limiting examples of synthetic aldehyde fragrances include 2-methyl undecanal, citral, hexyl cinnamic aldehyde, isocycolcitral, lilial, and 10-undecenal. Some non-limiting examples of synthetic ketone fragrances include cashmeran, .alpha.-ionone, isocyclemone E, koavone, muscone, and tonalide. Some non-limiting examples of synethetic ester fragrances include benzyl acetate, 4-t-butylcyclohexyl acetate (cis and trans), cedryl acetate, cyclacet, isobornyl acetate, and .alpha.-terpinyl acetate (R=acetyl). Some non-limiting examples of synthetic lactone fragrances include coumarin, jasmine lactone, muskalactone, and peach aldehyde. Some non-limiting examples of synthetic ether fragrances include ambroxan, anther, and galaxolide. Some non-limiting examples of synthetic nitrile fragrances include cinnamonitrile and gernonitrile. Finally, some non-limiting examples of synthetic polyfunctional fragrances include amyl salicylate, isoeugenol, hedione, heliotropine, lyral, and vanillin.

In some embodiments, the body care compositions comprise one or more foaming agents. The foaming agents include, for example, sodium lauryl sulfate, sodium lauroyl sarcosine, sodium alkyl sulfosuccinates, sodium coconut oil fatty acid monoglycerol sulfonates, sodium alpha.-olefin sulfonates, N-acylamino acid salts such as N-acyl glutamate, 2-alkyl-N-carboxymethyl-N-hydroxyethylimidazolinium betaine, maltitol fatty acid esters, sucrose fatty acid esters, polyglycerol fatty acid esters, fatty acid diethanolamides, polyoxyethylene sorbitan monostearate, polyoxyethylene hydrogenated castor oil and polyoxyethylene fatty acid esters. These foaming agents are usable either alone or in combination of two or more of them.

In some embodiments, the body care compositions comprise one or more tanning agents. Suitable tanning agents include, without limitation, alpha-hydroxy aldehydes and ketones, glyceraldehyde and related alcohol aldehydes, various indoles, imidazoles and derivatives thereof, and various approved pigmentation agents. Other suitable tanning agents include, without limitation, methyl glyoxal, glycerol aldehyde, erythrulose, alloxan, 2,3-dihydroxysuccindialdehyde, 2,3-dimethoxysuccindialdehyde, 2-amino-3-hydroxy-succindialdehyde and 2-benzylamino-3-hydroxysuccindialdehyde.

In some embodiments, the body care compositions comprise one or more astringents. Suitable astringents include, without limitation, aluminum citrate, aluminum lactate, extracts of birch, extracts of coffee, extracts of evening primrose, extracts of grape, extracts of henna, extracts of ivy, extracts of lemon, extracts of witch hazel, ammonium and potassium alum, aluminum triphosphate, aluminum glycinate and aluminum phenolsulfate, alcloxa, aldioxa, aluminum stearate, aluminum sulfate and aluminum citrate, sodium aluminum phosphate, sodium alum, sodium aluminum chlorohydroxy lactate, calcium lactate, calcium chloride, calcium sulfate hydrate, sodium aluminum lactate, zinc acetate, zinc chloride, zinc sulfate, zinc lactate, zinc zeolite, zinc phenolsulfonate, and combinations thereof.

In some embodiments, the body care compositions comprise one or more antiseptics. Suitable antiseptics include, without limitation, methyl, ethyl, propyl, or butyl ester of p-oxybenzoic acid, phenoxyethanol, o-phenylphenol, dehydroacetic acid, or salts thereof, p-cresol, m-cresol, o-chlor-m-xylenol, peppermint oil, Echinacea, bloodroot, cayenne, tea tree oil, wild bergamont, chaparral, stinging metal, bay, myrrh, rhatany bark, toothache tree, calendula, chamomile, mupirocin, neomycin sulfate, bacitracin, polymyxin B, 1-ofloxacin, tetracyclines (chlortetracycline hydrochloride, oxytetracycline hydrochloride and tetrachcycline hydrochoride), clindamycin phsphate, gentamicin sulfate, benzalkonium chloride, benzethonium chloride, hexylresorcinol, methylbenzethonium chloride, phenol, quaternary ammonium compounds, triclocarbon, triclosan, and tea tree oil.

In some embodiments, the body care compositions comprise one or more deodorants or antiperspirants. Suitable antiperspirants and deodorants include, without limitation, zinc salts such as zinc sulfate and zinc chloride, glycinates such as aluminum zirconium glycinate, aluminum chlorohydrate, aluminum zirconium tetrachlorohydrex, zinc carbonate, orthophenylphenol, and quaternary ammonium compounds such as dimethyl benzyl ammonium chloride and hexamethonium chloride.

In some embodiments, the body care compositions comprise one or more lightening agents. Examples of skin lighteners include, without limitation, hydroquinone, kojic acid, licorice or its derivatives, ascorbic acid or its derivatives, arbutin, bearberry extract, Glycyrrhiza glabra and its derivatives, Chlorella vulgaris extract, perilla extract, coconut fruit extract, and/or other depigmenting agents.

In some embodiments, the body care compositions comprise one or more biocides. Examples of biocides include, without limitation, triclosan, 3,4,4'-trichlorocarbanilide (triclocarban); 3,4,4'-trifluoromethyl-4,4'-dichlorocarbanilide (cloflucarban); 5-chloro-2-methyl-4-isothiazolin-3-one; iodopropynlbutylcarbamate; 8-hydroxyquinoline; 8-hydroxyquinoline citrate; 8-hydroxyquinoline sulfate; 4-chloro-3,5-xylenol(chloroxylenol); 2-bromo-2-nitropropane-1,3-diol; diazolidinyl urea; butoconazole; nystatin; terconazole; nitrofurantoin; phenazopyridine; acyclovir; clortrimazole; chloroxylenol; chlorhexidine; miconazole; terconazole; butylparaben; ethylparaben; methylparaben; methylchloroisothiazoline; methylisothiazoline; a mixture of 1,3-bis(hydroxymethyl)-5,5-dimethylhydantoin and 3-iodo-2-propynyl butyl carbamate; oxyquinoline; EDTA; tetrasodium EDTA; p-hydroxyl benzoic acid ester; alkyl pyridinum compounds; coco phosphatidyl PG-dimonium chloride; chlorhexidine gluconate; chlorhexidine digluconate; chlorhexidine acetate; chlorhexidine isethionate; chlorhexidine hydrochloride; benzalkonium chloride; benzethonium chloride; polyhexamethylene biguanide; and mixtures thereof.

Other ingredients that are typically used in body care compositions, such as skin care or hair care products, may be included.

### Flavored Products and Related Uses

In at least another aspect, the disclosure provides uses of the coolness-modifying composition of the preceding aspects and embodiments thereof to enhance a cooling sensation of a flavored product, such as to enhance the cooling sensation of a cooling compound (such as menthol, cubebol, or other cooling compounds set forth above) in a flavored product. In related aspects, the disclosure provides methods for enhancing a cooling sensation of a flavored product, the method comprising introducing a coolness-modifying composition of the preceding aspects and embodiments thereof to a flavored product.

In connection with these aspects, the flavored product can be any flavored product according to the aspects and embodiments set forth below. In some embodiments, the flavored product is a beverage product, such as enhanced sparkling beverages, colas, lemon-lime flavored sparkling beverages, orange flavored sparkling beverages, grape flavored sparkling beverages, strawberry flavored sparkling beverages, pineapple flavored sparkling beverages, ginger-ales, root beers, fruit juices, fruit-flavored juices, juice drinks, nectars, vegetable juices, vegetable-flavored juices, sports drinks, energy drinks, enhanced water drinks, enhanced water with vitamins, near water drinks, coconut waters, tea type drinks, coffees, cocoa drinks, beverages containing milk components, beverages containing cereal extracts and smoothies. In some other embodiments, the flavored product is a food product, such as a packaged meals, canned foods, meal replacement products, and the like. In some embodiments, the flavored product is an oral care product, such as a toothpaste, a mouthwash, or a teeth-whitening composition, such as a composition disclosed in U.S. Patent Application Pub. No. 2019/0167545.

The flavored product can be in any suitable physical form. For example, in some embodiments, the flavored composition can be in the form of a solid, such as a powder. In a further such embodiment, the flavored product is a toothpowder, such as a toothpowder for whitening teeth, removing stains (e.g., tobacco stains), and the like. In some other embodiments, the flavored product can be a paste or a slurry, such as a tooth paste. In some other embodiments, the flavored product can be in the form of a cream or gel, such as a tooth gel, a skin cream or tooth cream. In some other embodiments, the flavored product can be a liquid suspension or solution, for example, with an aqueous carrier. The flavored product can, in some embodiments, be in the form of an emulsion, such as a microemulsion or nanoemulsion, where a surfactant, emulsifier, and the like can be present. In some embodiments, the flavored product is a personal care, such as a product for application onto skin, face, lips, gums, mouth, and the like. Such personal products can be for any suitable use, such as skin whitening, teeth whitening, and the like.

In certain aspects, the disclosure provides flavored products comprising any compositions of the preceding aspects or embodiments thereof. In some embodiments, the flavored products are beverage products, such as soda, flavored water, tea, and the like. In some other embodiments, the flavored products are food products, such as yogurt.

In embodiments where the flavored product is a beverage, the beverage may be selected from the group consisting of enhanced sparkling beverages, colas, lemon-lime flavored sparkling beverages, orange flavored sparkling beverages, grape flavored sparkling beverages, strawberry flavored sparkling beverages, pineapple flavored sparkling beverages, ginger-ales, root beers, fruit juices, fruit-flavored juices, juice drinks, nectars, vegetable juices, vegetable-flavored juices, sports drinks, energy drinks, enhanced water drinks, enhanced water with vitamins, near water drinks, coconut waters, tea type drinks, coffees, cocoa drinks, beverages containing milk components, beverages containing cereal extracts and smoothies. In some embodiments, the beverage may be a soft drink.

In some embodiments, the flavored product is an oral care product. By "oral care product" is meant a personal care product or other product, which in the ordinary course of usage, is not intentionally swallowed for purposes of systemic administration of particular therapeutic agents, but is rather retained in the oral cavity for a time sufficient to contact substantially all of the dental surfaces and/or oral tissues for purposes of oral activity. The oral care composition may be in various forms including toothpaste, dentifrice, tooth gel, subgingival gel, mouthrinse, mousse, foam, mouthspray, lozenge, chewable tablet, chewing gum or denture product. The oral care composition may also be incorporated onto strips or films for direct application or attachment to oral surfaces.

In some embodiments, the flavored product is a pharmaceutical product, such as a product designed to carry or deliver certain active pharmaceutical ingredients (APIs). Such APIs can be over-the-counter (OTC) drugs, such as acetaminophen and other OTC cough and cold medications. In some embodiments, the API is a prescription-based drug.

In certain embodiments of any aspects and embodiments set forth herein that refer to a flavored product, the flavored product is a non-naturally-occurring product, such as a packaged food or beverage product.

Further non-limiting examples of food and beverage products or formulations include sweet coatings, frostings, or glazes for such products or any entity included in the Soup category, the Dried Processed Food category, the Beverage category, the Ready Meal category, the Canned or Preserved Food category, the Frozen Processed Food category, the Chilled Processed Food category, the Snack Food category, the Baked Goods category, the Confectionery category, the Dairy Product category, the Ice Cream category, the Meal Replacement category, the Pasta and Noodle category, and the Sauces, Dressings, Condiments category, the Baby Food category, and/or the Spreads category.

In general, the Soup category refers to canned/preserved, dehydrated, instant, chilled, UHT and frozen soup. For the purpose of this definition soup(s) means a food prepared from meat, poultry, fish, vegetables, grains, fruit and other ingredients, cooked in a liquid which may include visible pieces of some or all of these ingredients. It may be clear (as a broth) or thick (as a chowder), smooth, pureed or chunky, ready-to-serve, semi-condensed or condensed and may be served hot or cold, as a first course or as the main course of a meal or as a between meal snack (sipped like a beverage). Soup may be used as an ingredient for preparing other meal components and may range from broths (consommé) to sauces (cream or cheese-based soups).

The Dehydrated and Culinary Food Category usually means: (i) Cooking aid products such as: powders, granules, pastes, concentrated liquid products, including concentrated bouillon, bouillon and bouillon like products in pressed cubes, tablets or powder or granulated form, which are sold separately as a finished product or as an ingredient within a product, sauces and recipe mixes (regardless of technology); (ii) Meal solutions products such as: dehydrated and freeze dried soups, including dehydrated soup mixes, dehydrated instant soups, dehydrated ready-to-cook soups, dehydrated or ambient preparations of readymade dishes, meals and single serve entrees including pasta, potato and rice dishes; and (iii) Meal embellishment products such as: condiments, marinades, salad dressings, salad toppings, dips, breading, batter mixes, shelf stable spreads, barbecue sauces, liquid recipe mixes, concentrates, sauces or sauce mixes, including recipe mixes for salad, sold as a finished product or as an ingredient within a product, whether dehydrated, liquid or frozen.

The Beverage category usually means beverages, beverage mixes and concentrates, including but not limited to, carbonated and non-carbonated beverages, alcoholic and nonalcoholic beverages, ready to drink beverages, liquid concentrate formulations for preparing beverages such as sodas, and dry powdered beverage precursor mixes. The Beverage category also includes the alcoholic drinks, the soft drinks, sports drinks, isotonic beverages, and hot drinks. The alcoholic drinks include, but are not limited to beer, cider/perry, FABs, wine, and spirits. The soft drinks include, but are not limited to carbonates, such as colas and non-cola carbonates; fruit juice, such as juice, nectars, juice drinks and fruit flavored drinks; bottled water, which includes sparkling water, spring water and purified/table water; functional drinks, which can be carbonated or still and include sport, energy or elixir drinks; concentrates, such as liquid and powder concentrates in ready to drink measure. The drinks, either hot or cold, include, but are not limited to coffee or ice coffee, such as fresh, instant, and combined coffee; tea or ice tea, such as black, green, white, oolong, and flavored tea; and other drinks including flavor-, malt- or plant-based powders, granules, blocks or tablets mixed with milk or water.

The Snack Food category generally refers to any food that can be a light informal meal including, but not limited to Sweet and savory snacks and snack bars. Examples of snack food include, but are not limited to fruit snacks, chips/crisps, extruded snacks, tortilla/com chips, popcorn, pretzels, nuts and other sweet and savory snacks. Examples of snack bars include, but are not limited to granola/muesli bars, breakfast bars, energy bars, fruit bars and other snack bars.

The Baked Goods category generally refers to any edible product the process of preparing which involves exposure to heat or excessive sunlight. Examples of baked goods include, but are not limited to bread, buns, cookies, muffins, cereal, toaster pastries, pastries, waffles, tortillas, biscuits, pies, bagels, tarts, quiches, cake, any baked foods, and any combination thereof.

The Ice Cream category generally refers to frozen dessert containing cream and sugar and flavoring. Examples of ice cream include, but are not limited to: impulse ice cream; take-home ice cream; frozen yoghurt and artisanal ice cream; soy, oat, bean (e.g., red bean and mung bean), and rice-based ice creams.

The Confectionery category generally refers to edible product that is sweet to the taste. Examples of confectionery include, but are not limited to candies, gelatins, chocolate confectionery, sugar confectionery, gum, and the likes and any combination products.

The Meal Replacement category generally refers to any food intended to replace the normal meals, particularly for people having health or fitness concerns. Examples of meal replacement include, but are not limited to slimming products and convalescence products.

The Ready Meal category generally refers to any food that can be served as meal without extensive preparation or processing. The ready meal includes products that have had recipe "skills" added to them by the manufacturer, resulting in a high degree of readiness, completion and convenience. Examples of ready meal include, but are not limited to canned/preserved, frozen, dried, chilled ready meals; dinner mixes; frozen pizza; chilled pizza; and prepared salads.

The Pasta and Noodle category includes any pastas and/or noodles including, but not limited to canned, dried and chilled/fresh pasta; and plain, instant, chilled, frozen and snack noodles.

The Canned/Preserved Food category includes, but is not limited to canned/preserved meat and meat products, fish/seafood, vegetables, tomatoes, beans, fruit, ready meals, soup, pasta, and other canned/preserved foods.

The Frozen Processed Food category includes, but is not limited to frozen processed red meat, processed poultry, processed fish/seafood, processed vegetables, meat substitutes, processed potatoes, bakery products, desserts, ready meals, pizza, soup, noodles, and other frozen food.

The Dried Processed Food category includes, but is not limited to rice, dessert mixes, dried ready meals, dehydrated soup, instant soup, dried pasta, plain noodles, and instant noodles. The Chill Processed Food category includes, but is not limited to chilled processed meats, processed fish/seafood products, lunch kits, fresh cut fruits, ready meals, pizza, prepared salads, soup, fresh pasta and noodles.

The Sauces, Dressings and Condiments category includes, but is not limited to tomato pastes and purees, bouillon/stock cubes, herbs and spices, monosodium glutamate (MSG), table sauces, soy based sauces, pasta sauces, wet/cooking sauces, dry sauces/powder mixes, ketchup, mayonnaise, mustard, salad dressings, vinaigrettes, dips, pickled products, and other sauces, dressings and condiments.

The Baby Food category includes, but is not limited to milk- or soybean-based formula; and prepared, dried and other baby food.

The Spreads category includes, but is not limited to jams and preserves, honey, chocolate spreads, nut based spreads, and yeast based spreads.

The Dairy Product category generally refers to edible product produced from mammal's milk. Examples of dairy product include, but are not limited to drinking milk products, cheese, yoghurt and sour milk drinks, and other dairy products.

Additional examples for flavored products, particularly food and beverage products or formulations, are provided as follows. Exemplary ingestible compositions include one or more confectioneries, chocolate confectionery, tablets, countlines, bagged selflines/softlines, boxed assortments, standard boxed assortments, twist wrapped miniatures, seasonal chocolate, chocolate with toys, alfajores, other chocolate confectionery, mints, standard mints, power mints, boiled sweets, pastilles, gums, jellies and chews, toffees, caramels and nougat, medicated confectionery, lollipops, liquorice, other sugar confectionery, bread, packaged/industrial bread, unpackaged/artisanal bread, pastries, cakes, packaged/industrial cakes, unpackaged/artisanal cakes, cookies, chocolate coated biscuits, sandwich biscuits, filled biscuits, savory biscuits and crackers, bread substitutes, breakfast cereals, rte cereals, family breakfast cereals, flakes, muesli, other cereals, children's breakfast cereals, hot cereals, ice cream, impulse ice cream, single portion dairy ice cream, single portion water ice cream, multi-pack dairy ice cream, multi-pack water ice cream, take-home ice cream, take-home dairy ice cream, ice cream desserts, bulk ice cream, take-home water ice cream, frozen yoghurt, artisanal ice cream, dairy products, milk, fresh/pasteurized milk, full fat fresh/pasteurized milk, semi skimmed fresh/pasteurized milk, long-life/uht milk, full fat long life/uht milk, semi skimmed long life/uht milk, fat-free long life/uht milk, goat milk, condensed/evaporated milk, plain condensed/evaporated milk, flavored, functional and other condensed milk, flavored milk drinks, dairy only flavored milk drinks, flavored milk drinks with fruit juice, soy milk, sour milk drinks, fermented dairy drinks, coffee whiteners, powder milk, flavored powder milk drinks, cream, cheese, processed cheese, spreadable processed cheese, unspreadable processed cheese, unprocessed cheese, spreadable unprocessed cheese, hard cheese, packaged hard cheese, unpackaged hard cheese, yoghurt, plain/natural yoghurt, flavored yoghurt, fruited yoghurt, probiotic yoghurt, drinking yoghurt, regular drinking yoghurt, probiotic drinking yoghurt, chilled and shelf-stable desserts, dairy-based desserts, soy-based desserts, chilled snacks, fromage frais and quark, plain fromage frais and quark, flavored fromage frais and quark, savory fromage frais and quark, sweet and savory snacks, fruit snacks, chips/crisps, extruded snacks, tortilla/corn chips, popcorn, pretzels, nuts, other sweet and savory snacks, snack bars, granola bars, breakfast bars, energy bars, fruit bars, other snack bars, meal replacement products, slimming products, convalescence drinks, ready meals, canned ready meals, frozen ready meals, dried ready meals, chilled ready meals, dinner mixes, frozen pizza, chilled pizza, soup, canned soup, dehydrated soup, instant soup, chilled soup, hot soup, frozen soup, pasta, canned pasta, dried pasta, chilled/fresh pasta, noodles, plain noodles, instant noodles, cups/bowl instant noodles, pouch instant noodles, chilled noodles, snack noodles, canned food, canned meat and meat products, canned fish/seafood, canned vegetables, canned tomatoes, canned beans, canned fruit, canned ready meals, canned soup, canned pasta, other canned foods, frozen food, frozen processed red meat, frozen processed poultry, frozen processed fish/seafood, frozen processed vegetables, frozen meat substitutes, frozen potatoes, oven baked potato chips, other oven baked potato products, non-oven frozen potatoes, frozen bakery products, frozen desserts, frozen ready meals, frozen pizza, frozen soup, frozen noodles, other frozen food, dried food, dessert mixes, dried ready meals, dehydrated soup, instant soup, dried pasta, plain noodles, instant noodles, cups/bowl instant noodles, pouch instant noodles, chilled food, chilled processed meats, chilled fish/seafood products, chilled processed fish, chilled coated fish, chilled smoked fish, chilled lunch kit, chilled ready meals, chilled pizza, chilled soup, chilled/fresh pasta, chilled noodles, oils and fats, olive oil, vegetable and seed oil, cooking fats, butter, margarine, spreadable oils and fats, functional spreadable oils and fats, sauces, dressings and condiments, tomato pastes and purees, bouillon/stock cubes, stock cubes, gravy granules, liquid stocks and fonds, herbs and spices, fermented sauces, soy based sauces, pasta sauces, wet sauces, dry sauces/powder mixes, ketchup, mayonnaise, regular mayonnaise, mustard, salad dressings, regular salad dressings, low fat salad dressings, vinaigrettes, dips, pickled products, other sauces, dressings and condiments, baby food, milk formula, standard milk formula, follow-on milk formula, toddler milk formula, hypoallergenic milk formula, prepared baby food, dried baby food, other baby food, spreads, jams and preserves, honey, chocolate spreads, nut-based spreads, and yeast-based spreads. Exemplary ingestible compositions also include confectioneries, bakery products, ice creams, dairy products, sweet and savory snacks, snack bars, meal replacement products, ready meals, soups, pastas, noodles, canned foods, frozen foods, dried foods, chilled foods, oils and fats, baby foods, or spreads or a mixture thereof. Exemplary ingestible compositions also include breakfast cereals, sweet beverages or solid or liquid concentrate compositions for preparing beverages, ideally so as to enable the reduction in concentration of previously known saccharide sweeteners, or artificial sweeteners.

Some embodiments provide a chewable composition that may or may not be intended to be swallowed. In some embodiments, the chewable composition may be gum, chewing gum, sugarized gum, sugar-free gum, functional gum, bubble gum including compounds as disclosed and described herein, individually or in combination.

In some embodiments, the coolness-modifying compositions as disclosed and described herein, individually or in combination, may be provided in a flavoring concentrate formulation, e.g., suitable for subsequent processing to produce a ready-to-use (i.e., ready-to-serve) product. By "a flavoring concentrate formulation", it is meant a formulation which should be reconstituted with one or more diluting medium to become a ready-to-use composition. The term "ready-to-use composition" is used herein interchangeably with "ingestible composition", which denotes any substance that, either alone or together with another substance, can be taken by mouth whether intended for consumption or not. In one embodiment, the ready-to-use composition includes a composition that can be directly consumed by a human or animal. The flavoring concentrate formulation is typically used by mixing with or diluted by one or more diluting medium, e.g., any consumable or ingestible ingredient or product, to impart or modify one or more flavors to the diluting medium. Such a use process is often referred to as reconstitution. The reconstitution can be conducted in a household setting or an industrial setting. For example, a frozen fruit juice concentrate can be reconstituted with water or other aqueous medium by a consumer in a kitchen to obtain the ready-to-use fruit juice beverage. In another example, a soft drink syrup concentrate can be reconstituted with water or other aqueous medium by a manufacturer in large industrial scales to produce the ready-to-use soft drinks. Since the flavoring concentrate formulation has the flavoring agent or flavor modifying agent in a concentration higher than the ready-to-use composition, the flavoring concentrate formulation is typically not suitable for being consumed directly without reconstitution. There are many benefits of using and producing a flavoring concentrate formulation. For example, one benefit is the reduction in weight and volume for transportation as the flavoring concentrate formulation can be reconstituted at the time of usage by the addition of suitable solvent, solid or liquid.

The flavored products set forth according to any of the foregoing embodiments, also include, in certain embodiments, one or more additional flavor-modifying compounds, such as compounds that enhance sweetness (e.g., hesperetin, naringenin, glucosylated steviol glycosides, etc.), compounds that block bitterness, compounds that enhance umami, compounds that reduce sourness, compounds that enhance saltiness, compounds that enhance a cooling effect, or any combinations of the foregoing.

**In** certain embodiments of any aspects and embodiments set forth herein that refer to a sweetening or flavoring concentrate, the sweetening or flavoring concentrate is a non-naturally-occurring product, such as a composition specifically manufactured for the production of a flavored product, such as food or beverage product.

In one embodiment, the flavoring concentrate formulation comprises i) compounds as disclosed and described herein, individually or in combination; ii) a carrier; and iii) optionally at least one adjuvant. The term "carrier" denotes a usually inactive accessory substance, such as solvents, binders, or other inert medium, which is used in combination with the present compound and one or more optional adjuvants to form the formulation. For example, water or starch can be a carrier for a flavoring concentrate formulation. In some embodiments, the carrier is the same as the diluting medium for reconstituting the flavoring concentrate formulation; and in other embodiments, the carrier is different from the diluting medium. The term "carrier" as used herein includes, but is not limited to, ingestibly acceptable carrier.

The term "adjuvant" denotes an additive which supplements, stabilizes, maintains, or enhances the intended function or effectiveness of the active ingredient, such as the compound of the present invention. In one embodiment, the at least one adjuvant comprises one or more flavoring agents. The flavoring agent may be of any flavor known to one skilled in the art or consumers, such as the flavor of chocolate, coffee, tea, mocha, French vanilla, peanut butter, chai, or combinations thereof. In another embodiment, the at least one adjuvant comprises one or more sweeteners. The one or more sweeteners can be any of the sweeteners described in this application. In another embodiment, the at least one adjuvant comprises one or more ingredients selected from the group consisting of a emulsifier, a stabilizer, an antimicrobial preservative, an antioxidant, vitamins, minerals, fats, starches, protein concentrates and isolates, salts, and combinations thereof. Examples of emulsifiers, stabilizers, antimicrobial preservatives, antioxidants, vitamins, minerals, fats, starches, protein concentrates and isolates, and salts are described in U.S. Pat. No. 6,468,576.

In one embodiment, the present flavoring concentrate formulation can be in a form selected from the group consisting of liquid including solution and suspension, solid, foamy material, paste, gel, cream, and a combination thereof, such as a liquid containing certain amount of solid contents. In one embodiment, the flavoring concentrate formulation is in form of a liquid including aqueous-based and nonaqueous-based. In some embodiments, the present flavoring concentrate formulation can be carbonated or non-carbonated.

The flavoring concentrate formulation may further comprise a freezing point depressant, nucleating agent, or both as the at least one adjuvant. The freezing point depressant is an ingestibly acceptable compound or agent which can depress the freezing point of a liquid or solvent to which the compound or agent is added. That is, a liquid or solution containing the freezing point depressant has a lower freezing point than the liquid or solvent without the freezing point depressant. In addition to depress the onset freezing point, the freezing point depressant may also lower the water activity of the flavoring concentrate formulation. The examples of the freezing point depressant include, but are not limited to, carbohydrates, oils, ethyl alcohol, polyol, e.g., glycerol, and combinations thereof. The nucleating agent denotes an ingestibly acceptable compound or agent which is able to facilitate nucleation. The presence of nucleating agent in the flavoring concentrate formulation can improve the mouthfeel of the frozen Blushes of a frozen slush and to help maintain the physical properties and performance of the slush at freezing temperatures by increasing the number of desirable ice crystallization centers. Examples of nucleating agents include, but are not limited to, calcium silicate, calcium carbonate, titanium dioxide, and combinations thereof.

In one embodiment, the flavoring concentrate formulation is formulated to have a low water activity for extended shelf life. Water activity is the ratio of the vapor pressure of water in a formulation to the vapor pressure of pure water at the same temperature. In one embodiment, the flavoring concentrate formulation has a water activity of less than about 0.85. In another embodiment, the flavoring concentrate formulation has a water activity of less than about 0.80. In another embodiment, the flavoring concentrate formulation has a water activity of less than about 0.75.

### Body Care Products and Related Uses

In at least another aspect, the disclosure provides uses of the coolness-modifying composition described above to enhance a cooling sensation of a body care product. In some embodiments, the body care product comprises a cooling compound (for example, menthol, cubebol, or a combination thereof). In some embodiments, the body care product is a shampoo, a body wash, a soap, an exfoliating scrub, a moisturizer, a facial serum, an antiperspirant, a deodorant, a body lotion, a body oil, a sunscreen, or a bronzer.

In at least another aspect, the disclosure provides methods enhancing a cooling sensation of a body care product, the method comprising combining the coolness-modifying composition described above with a cooling compound (for example, menthol, cubebol, or a combination thereof) in a body care product. In some embodiments, the body care product is a shampoo, a body wash, a soap, an exfoliating scrub, a moisturizer, a facial serum, an antiperspirant, a deodorant, a body lotion, a body oil, a sunscreen, or a bronzer.

In at least another aspect, the disclosure provides body care products comprising a coolness-modifying composition described above and a cooling compound (for example, menthol, cubebol, or a combination thereof). In some embodiments, the body care product is a shampoo, a body wash, a soap, an exfoliating scrub, a moisturizer, a facial serum, an antiperspirant, a deodorant, a body lotion, a body oil, a sunscreen, or a bronzer.

In some embodiments, the body care product is a personal care or cosmetic product. Personal care and cosmetic products include products that can be applied to the skin, hair and nails, either as leave on or rinse off product. In the context of the present disclosure "rinse-off" means that the intended product use includes application to skin and/or hair followed by rinsing or wiping the product from the skin or hair within a few seconds to minutes of the application step. Personal care and cosmetic products include powders, creams, emulsions, lotions, gels and oils for the skin (face, hands, feet, and the like), tinted bases (liquids and pastes) and liquid impregnated tissues; products for applying and removing make-up from the face and eyes; hair care products including hair tints and bleaches; products for waving, straightening, setting and fixing hair; shaving products including creams, foams mousses and depilatory products; sun bathing products and products for tanning without the sun; deodorant and antiperspirant products.

In some embodiments a personal care or cosmetic product is selected from the group consisting of a shaving aid, a shampoo, a hair-conditioner product, a leave-on-skin-care product, a skin cleansing or washing product (such as a rinse-off skin cleansing or washing product), a moist tissue and a body spray, deodorant or antiperspirant. Shaving aids specifically include foams, gels, creams and bars (reference can be made for example to U.S. Pat. No. 7,069,658, U.S. Pat. No. 6,944,952, U.S. Pat. No. 6,594,904, U.S. Pat. No. 6,182,365, U.S. Pat. No. 6,185,822, U.S. Pat. No. 6,298,558 and U.S. Pat. No. 5,113,585). Shampoos and hair conditioners specifically include two-in-one shampoos and shampoos especially formulated for dry or greasy hair or containing additives such as antidandruff agents. Hair conditioners can be rinse off or leave on hair conditioners also included are hair tonics, bleaches colorants, setting and styling products. Reference can be made for example to U.S. Pat. No. 6,162,423, U.S. Pat. No. 5,968,286, U.S. Pat. No. 5,935,561, U.S. Pat. No. 5,932,203, U.S. Pat. No. 5,837,661, U.S. Pat. No. 5,776,443, U.S. Pat. No. 5,756,436, U.S. Pat. No. 5,661,118, U.S. Pat. No. 5,618,523. Leave-on-skin-care products include Eau de Parfum, Eau de toilette, colognes, moist tissues, body sprays, deodorants and antiperspirants. Skin washing products specifically include beauty and hygiene bar soaps, shower gels, liquid soaps, body washes, exfoliating gels and pastes (reference can be made for example to U.S. Pat. No. 3,697,644; U.S. Pat. No. 4,065,398; U.S. Pat. No. 4,387,040). Moist tissues (wipes) specifically include skin cleansing wipes, baby wipes, make-up removal wipes and skin refreshing wipes (reference can be made for example to U.S. Pat. No. 4,775,582; WO 02/07701; WO 2007/069214 and WO 95/16474). Body sprays, deodorants and antiperspirants specifically include sticks, liquid roll-on applicators and pressurized sprays.

### EXAMPLES

To further illustrate this invention, the following examples are included. The examples should not, of course, be construed as specifically limiting the invention. Variations of these examples within the scope of the claims are within the purview of one skilled in the art and are considered to fall within the scope of the invention as described, and claimed herein. The reader will recognize that the skilled artisan, armed with the present disclosure, and skill in the art is able to prepare and use the invention without exhaustive examples.

### Example 1 - Toothpaste Formulation

Toothpaste compositions are made according to certain embodiments of the present disclosure. Table 1 below sets forth a list of ingredients (wt%) for inclusion in two control compositions and two test compositions using coolness-enhancing compounds.

**Table 1**

| | **Control 1** | **Control 2** | **Sample 1** | **Sample 2** |
|---|---|---|---|---|
| Opaque toothpaste base | 98.50% | 98.50% | 98.50% | 98.50% |
| Sodium Saccharin | 0.20% | 0.20% | 0.20% | 0.20% |
| Menthol | 0.30% | 0.40% | 0.30% | 0.30% |
| *trans-*2-methyl-2-butenal | 0.00% | 0.00% | 0.01% | 0.00% |
| 2-Isopropyl-5-methyl-2-hexenal | 0.00% | 0.00% | 0.00% | 0.01% |
| Water | 1.00% | 0.90% | 0.99% | 0.99% |

### Example 2 - Sensory Testing

Sensory testers (a panel of four oral care flavorists) were asked to perform the following tests: (1) brush for 1 minute with the control samples first; (2) give a score at different time intervals as mentioned below on 3 key measures (cooling, bitter, burning, and any other notes perceived); and (3) use the 0 to 5 scale as shown below with 0 indicating no impact/intensity, and 5 indicating extremely high impact/intensity. Table 2 shows the results.

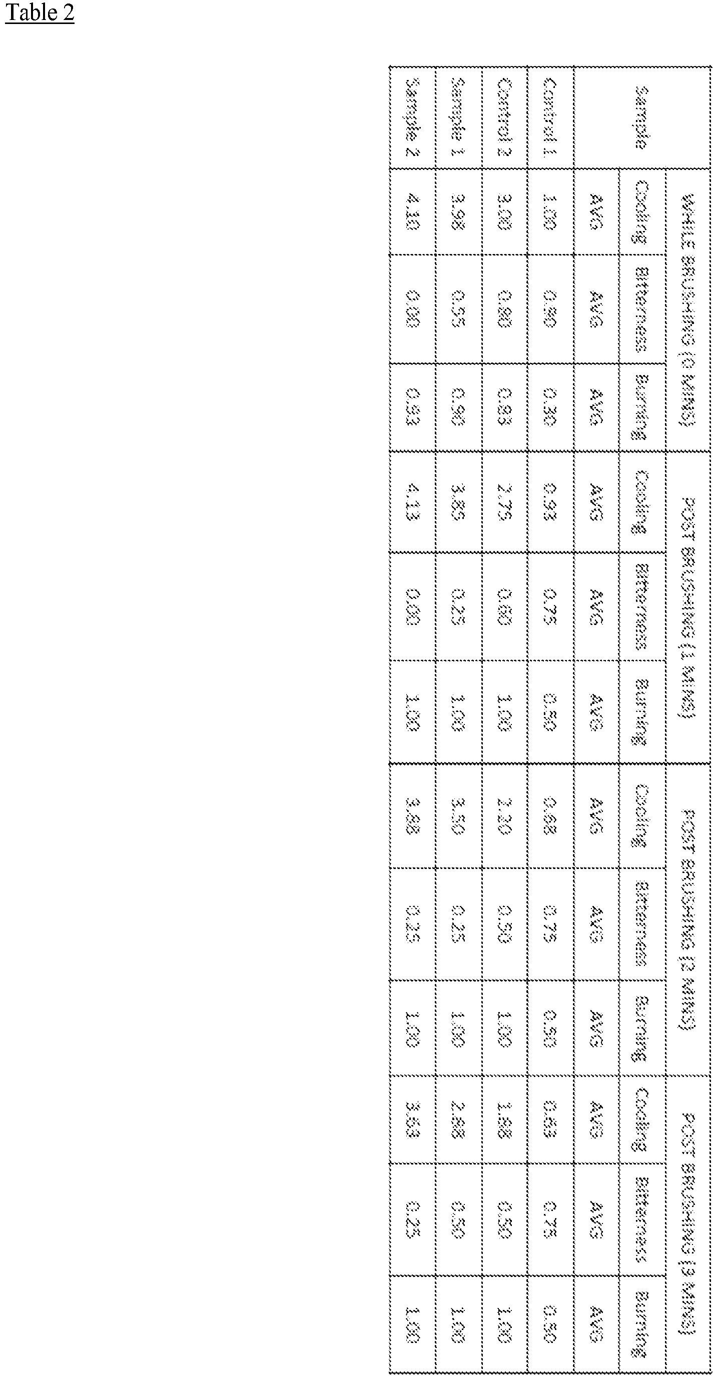

### Example 3 - Toothpaste Composite Base

Table 3 below shows sample ingredients of a toothpaste formulation of certain embodiments disclosed herein. Amounts are given in weight percent.

**Table 3**

| **TOOTHPASTE COMP. - BASE** | **Sample 1A** | **Sample 2A** |
|---|---|---|
| Polyethylene Glycol 400 | 2.00% | 2.00% |
| Xanthan Gum | 0.60% | 0.60% |
| Sorbitol 70% Solution | 50.00% | 50.00% |
| Sodium Fluoride | 0.22% | 0.22% |
| Sodium Benzoate | 0.20% | 0.20% |
| Water | 15.02% | 15.02% |
| Hydrated Silica (Tixosil 73) | 22.00% | 22.00% |
| Hydrated Silica (Tixosil 43) | 7.00% | 7.00% |
| Titanium Dioxide Ci77891 | 0.50% | 0.50% |
| Sodium Lauryl Sulfate | 1.25% | 1.25% |
| Mint Flavor | 1.00% | 1.00% |
| *trans*-2-methyl-2-butenal | 0.01% | 0.00% |
| 2-Isopropyl-5-methyl-2-hexenal | 0.00% | 0.01% |
| Sodium Saccharin | 0.20% | 0.20% |

### Example 4 - Mouthwash Composite Base

Tables 4 and 5 below show sample ingredients of a mouthwash formulation of certain embodiments disclosed herein. Amounts are given in weight percent.

**Table 4**

| **MOUTHWASH COMPOSITION** | **Sample 3** | **Sample 4** |
|---|---|---|
| Dissolvant PG | 10.00% | 10.00% |
| Sodium Fluoride | 0.02% | 0.02% |
| DI/Purified Water | 76.36% | 76.36% |
| Poloxamer 407 NF | 0.25% | 0.25% |
| Sorbitol 70% Solution | 8.00% | 8.00% |
| Sodium Saccharin | 0.02% | 0.02% |
| Sodium Benzoate | 0.06% | 0.06% |
| Mint Flavor | 0.20% | 0.20% |
| *trans-*2-methyl-2-butenal | 0.01% | 0.00% |
| 2-Isopropyl-5-methyl-2-hexenal | 0.00% | 0.01% |
| Glycerine NAT | 5.00% | 5.00% |
| Sucralose | 0.01% | 0.01% |
| Benzoic Acid | 0.07% | 0.07% |

**Table 5**

| **MOUTHWASH COMPOSITION** | **Sample 5** | **Sample 6** |
|---|---|---|
| Ethyl Alcohol 190 Proof | 15.00% | 15.00% |
| Mint Flavor | 0.20% | 0.20% |
| *trans-*2-methyl-2-butenal | 0.01% | 0.00% |
| 2-Isopropyl-5-methyl-2-hexenal | 0.00% | 0.01% |
| DI/Purified Water | 71.31% | 71.31% |
| Poloxamer 407 NF | 0.24% | 0.24% |
| Sodium Lauryl Sulfate | 0.04% | 0.04% |
| Sorbitol 70% Solution | 10.00% | 10.00% |
| Sodium Saccharin | 0.03% | 0.03% |
| Glycerine | 3.00% | 3.00% |
| Sodium Benzoate | 0.10% | 0.10% |
| Sucralose | 0.02% | 0.02% |
| Benzoic Acid | 0.05% | 0.05% |

### Example 5 - Cubebol Toothpaste Formulation

Toothpaste compositions are made according to certain embodiments of the present disclosure. Table 6 below sets forth a list of ingredients (wt%) for inclusion in two control compositions and two test compositions using coolness-enhancing compounds.

**Table 6**

| | **Sample 7** | **Sample 7** | **Sample 8** |
|---|---|---|---|
| Opaque toothpaste base | 98.50% | 98.50% | 98.50% |
| Sodium Saccharin | 0.20% | 0.20% | 0.20% |
| Cubebol | 0.01% | 0.01% | 0.01% |
| *trans*-2-methyl-2-butenal | 0.00% | 0.01% | 0.00% |
| 2-Isopropyl-5-methyl-2-hexenal | 0.00% | 0.00% | 0.01% |
| Water | 1.29% | 1.28% | 1.28% |

### Example 6 - Sensory Testing

Sensory testers (a panel of four oral care flavorists) were asked to perform the following tests: (1) brush for 1 minute with the control samples first; (2) give a score at different time intervals as mentioned below on 3 key measures (cooling, bitter, burning, and any other notes perceived); and (3) use the 0 to 5 scale as shown below with 0 indicating no impact/intensity, and 5 indicating extremely high impact/intensity. Table 7 shows the results.

**Table 7**

| | S7 | S8 | S9 |
|---|---|---|---|
| While brushing | | | |
| Cooling level | 0.3 | 0.5 | 0.7 |
| Bitterness level | 0.6 | 0.2 | 0.2 |
| Burning Level | 0.0 | 0.0 | 0.0 |
| Other notes - please describe | | almond pleasant | herbal mint |

| 3 min after brushing | | | |
|---|---|---|---|
| Cooling level | 0.5 | 1.2 | 1.6 |
| Bitterness level | 1.0 | 0.5 | 0.5 |
| Burning Level | 0.0 | 0.1 | 0.1 |
| Other comments | | quite pleasant lasting cool develops over time. Clean mouthfeel long lasting cool. | quite pleasant lasting cool develops over time. Clean mouthfeel long lasting cool. |

### Example 7 - Shower Gel

A shower gel base was prepared having the ingredients and respective amounts shown in Table 8 (in wt%)

**Table 8**

| **Ingredient** | **Conc. (%)** |
|---|---|
| Deionised Water | 52.4 |
| Tetrasodium EDTA ¹⁾ | 0.1 |
| Sodium Benzoate | 0.5 |
| Propylene Glycol | 2.0 |
| Sodium C12-C15 Pareth Sulfate ²⁾ | 35.0 |
| Cocamidopropyl Betaine ³⁾ | 8.0 |
| Polyquaternium-7 ⁴⁾ | 0.2 |
| Citric Acid 40% aq | q.s. |
| Sodium Chloride | q.s. |
| **TOTAL** | **100.0** |

| | |
|---|---|
| 1) Edeta B Powder, BASF 2) Zetesol AO 328 U, Zschimmer & Schwarz 3) Tego-Betain F50, Evonik 4) Merquat 550, Lubrizol | |

The ingredients of the shower gel base (above) were mixed and solubilized separately by addition of one after the other, in the given sequence. The final pH of the shower gel base was adjusted with citric acid solution until the pH was in the range of 4.3 to 4.8. The final viscosity of the composition was adjusted with sodium chloride addition, to 3000 cPs (+/- 1500 cPs) Brookfield RV / Spindle#4 / Speed#20rpm.

The shower gel base was mixed with menthol and 2-isopropyl-5-methyl-2-hexenal in the relative amounts shown in Table 9 (in wt%).

**Table 9**

| | **Example 7A CONTROL** | **Example 7B TEST SAMPLE** |
|---|---|---|
| Perfumed Shower-Gel base (PGK15/005) | 99.68% | 99.68% |
| Menthol | 0.30% | 0.30% |
| 2-Isopropyl-5-methyl-2-hexenal | - | 0.02% |
| Water | 0.02% | - |
| **TOTAL** | **100** | **100** |

### Example 8 - Shower Gel Sensory Evaluation

A shower gel of Example 7A and 7B were subjected to sensory evaluation. A sample of 22 panelists each stepped into the shower and wet their whole body under running water. Then, they applied the shower gel onto their wet hand, and onto their whole body making circular motions and creating a good foam. Then, the flow of water was turned off. Cooling intensity at foaming was evaluated at this point. The panelists then rinsed their body thoroughly before stepping out of the shower and drying. Cooling intensity was evaluated at specified evaluation points. The panelists were asked to evaluate the cooling sensation on a scale from 1 to 10 at five different points in time: while showering, and at time intervals of 5 minutes, 15 minutes, 30 minutes, and 45 minutes after showering. Table 10 shows the mean evaluations of the 22 panelists at these points in time.

**Table 10**

| **Cooling intensity at various evaluation stages** | **Example 7A CONTROL** | **Example 7B TEST SAMPLE** |
|---|---|---|
| While washing under shower | 5.14 | 4.82 |
| On dry skin 5 min after showering | 4.72 | 4.83 |
| On dry skin 15 min after showering | 2.59 | 2.72 |
| On dry skin 30 min after showering | 1.04 | 1.13 |
| On dry skin 45 min after showering | 0.12 | 0.50 |

### Example 9 - Shampoo

A shampoo base was prepared having the ingredients and respective amounts shown in Table 11. To make phase A, the polymer JR-400 was dispersed in water. The remaining ingredients of phase A were mixed separately by addition of one after the other while mixing well after each adjunction. This pre-mix was added to the polymer dispersion and mixed for another 5 min. The ingredients of phase B and the premixed phase C were added (Monomuls 90L-12 was heated to melt in Texapon NSO IS) while agitating to mix. The ingredients of phase D and phase E were added while agitating. Thereafter, the pH was adjusted with citric acid solution to reach a pH ranging from 5.5 to 6.0.

**Table 11**

| | **Transparent shampoo Ingredients / INCI name** | **wt %** |
|---|---|---|
| A | Water deionized | 44.4 |
| | Polyquaternium-10 ¹⁾ | 0.3 |
| | Glycerin 85% ²⁾ | 1 |
| | DMDM Hydantoin ³⁾ | 0.2 |
| | | |
| B | Sodium Laureth Sulfate ⁴⁾ | 28 |
| | Cocamidopropyl Betaine ⁵⁾ | 3.2 |
| | Disodium Cocoamphodiacetate ⁶⁾ | 4 |
| | Ethoxy (20) Stearyl Alcohol ⁶⁾ | 1 |
| | | |
| C | Sodium Laureth Sulfate ⁴⁾ | 3 |
| | Glyceryl Laureate ⁷⁾ | 0.2 |
| | | |
| D | Water deionized | 1 |
| | Sodium Methylparaben ⁸⁾ | 0.1 |
| | | |
| E | Sodium Chloride 10% aqueous sol. | 15 |
| | Citric acid 10% | q.s. |
| | Perfume | 0.5 |
| | **T O T A L:** | **100** |

| | | |
|---|---|---|
| 1) Ucare Polymer JR-400, Noveon 2) Schweizerhall 3) Glydant, Lonza 4) Texapon NSO IS, Cognis 5) Tego Betain F 50, Evonik 6) Amphotensid GB 2009, Zschimmer & Schwarz 7) Monomuls 90 L-12, Gruenau 8) Nipagin Monosodium, NIPA | | |

The shampoo base was mixed with menthol and 2-isopropyl-5-methyl-2-hexenal in the relative amounts shown in Table 12.

**Table 12**

| | **Example 9A CONTROL** | **Example 9B SAMPLE** | **Example 9C SAMPLE** |
|---|---|---|---|
| Perfumed Transparent Shampoo (EDL 10-001) | 99.60% | 99.58% | 99.55% |
| Menthol | 0.40% | 0.40% | 0.40% |
| 2-Isopropyl-5-methyl-2-hexenal | - | 0.02% | 0.05% |
| **TOTAL** | **100** | **100** | **100** |

### Example 10 - Shampoo Sensory Evaluation

A shampoo of Examples 9A, 9B , and 9C were subjected to sensory evaluation. A sample of 17 panelists each wet their hair and washed thoroughly with foaming for at least 2 minutes using a normal amount of the shampoo provided. Then, each panelist rinsed the hair thoroughly, got out of the shower, and dried the body and hair using a towel. Cooling intensity was evaluated at specified evaluation points. The panelists were asked to evaluate the cooling sensation on a scale from 1 to 7 at five different points in time: during foaming, and at time intervals of 15 minutes, 30 minutes, one hour and two hours after showering. Table 13 shows the mean evaluations of the 17 panelists at these points in time.

**Table 13**

| **Stage of Cooling Evaluation** | **Example 9A CONTROL** | **Example 9B SAMPLE** | **Example 9C SAMPLE** |
|---|---|---|---|
| Foaming | 4.1 | 4.25 | 4.5 |
| 15 min after drying | 3.6 | 3.8 | 4.3 |
| 30 min after drying | 2.65 | 2.95 | 3.50 |
| 1 hour after drying | 2.15 | 2.2 | 2.4 |
| 2 hours after drying | 1.80 | 1.85 | 1.9 |

## Claims

1. Use of a coolness-enhancing compound to enhance a cooling sensation of a flavored product or a body care product, wherein the coolness-enhancing compound is selected from the group consisting of: (E/Z)-2-methyl-2-butenal, (E/Z)-2-isopropyl-5-methyl-2-hexenal, and any combination thereof.

2. The use of claim 1, wherein the coolness-enhancing compound is (E/Z)-2-methyl-2-butenal.

3. The use of claim 1, wherein the coolness-enhancing compound is (E/Z)-2-isopropyl-5-methyl-2-hexenal.

4. The use of any one of claims 1 to 3, wherein the flavored product or the body care product comprises a cooling substance.

5. The use of claim 4, wherein the cooling substance is (-)-menthol, N-ethyl-p-menthyl-3-carboxiamide, cubebol, isopulegol, 3-(L-menthoxy) propane-1,2-diol, cis/trans-p-menthane-3,8-diol, 3-(L-menthoxy)-2-methylpropane-1,2-diol, 2-[2-(p-menthan-3-yloxy)ethoxy]ethanol, menthoxyethanol, (1R,2R,4R)-1-(2-hydroxy-4-methylcyclohexyl)ethenone, (1R,2R,5R)-N-ethyl-5-methyl-2-(prop-1-en-2-yl)-cyclohexanecarboxamide, N-(3-hydroxy-4-methoxyphenyl)-2-isopropyl-5,5-dimethyl-cyclohexanecarboxamide, N-[4-(cyanomethyl)phenyl]-2-isopropyl-5,5-dimethyl-cyclohexanecarboxamide, N-(2-Hydroxy-2-phenylethyl)-2-isopropyl-5,5-dimethyl-cyclohexane-1-carboxamide, L-menthol lactate, (-)-menthyl ethylene glycol carbonate, (-)-menthone 1,2-glycerol ketal, D/L-menthone 1,2-glycerol ketal, (-)-menthyl 1-propylene glycol carbonate, (-)-menthyl 2-propylene glycol carbonate, D/L-menthyl 1-propylene glycol carbonate, D/L-menthyl 2-propylene glycol carbonate, (1R,2S,5R)-N-(4-methoxyphenyl)-5-methyl-2-(1-methylethyl)cyclohexanecarboxamide, menthyl ethylamido oxalate, (E)-3-benzo[1,3]dioxol-5-yl-N,N-diphenyl-2-propenamide, 3,4-methylenedioxycinnamic acid, N,N-diphenylamide, N,N-dimethyl (-)-menthyl succinimide, (1R,2S,5R)-N-(4-(carbamoylmethyl)phenyl)-menthylcarboxamide, (-)-menthyl pyrrolidone carboxylate, L-phenylephrine p-menthane carboxamide, (1R,2S,5R)-N-(4-(cyanomethyl)-phenyl)menthylcarboxamide, (1R,2S,5R)-N-(2-(pyridin-2-yl)ethyl)menthylcarboxamide, 6-isopropyl-3,9-dimethyl-1,4-dioxaspiro[4.5]decan-2-one, (1R,2R,4S)-dihydroumbellulol, N-ethyl-p-menthane-3-carboxamide, 2-isopropyl-N,2,3-trimethylbutyramide, ethyl 3-(p-menthane-3-carboxamido)acetate, N-ethyl-2,2-diisopropylbutanamide, N-(1,1-dimethyl-2-hydroxyethyl)-2,2-dimethylbutanamide, N-cyclopropyl-5-methyl-2-isopropyl-cyclohexanecarbonecarboxamide, (-)-menthyl acetoacetate, (-)-menthyl succinate, (-)-menthyl (S)-3-hydroxybutyrate, (-)-menthyl glutarate, 1-[2-hydroxyphenyl]-4-[2-nitrophenyl]-1,2,3,6-tetrahydropyrimidine-2-one, di-(-)-menthyl glutarate, N-(2-hydroxyethyl)-2,3-dimethyl-2-isopropylbutanamide, and aby combinations thereof.

6. The use of claim 5, wherein the cooling substance is selected from the group consisting of: (-)-menthol, N-ethyl-p-menthyl-3-carboxiamide, cubebol, and any combinations thereof.

7. The use of any one of claims 1 to 6, wherein the coolness-enhancing compound is used in a flavored product, and the flavored product comprises a high-intensity sweetener.

8. The use of claim 7, wherein the high-intensity sweetener is sodium saccharine, sucralose, or any combination thereof.

9. The use of any one of claims 1 to 8, wherein the flavored product comprises 4-amino-5,6-dimethyl-thieno[2,3-d]pyrimidin-2(1H)-one, or a salt thereof.

10. The use of any one of claims 1 to 9, wherein the flavored product further comprises a flavoring, such as mint flavoring.

11. The use of any one of claims 1 to 10, wherein the flavored product or the body care product further comprises a compound selected from the group consisting of:
N-ethyl-N-(thiophen-2-ylmethyl)-2-(p-tolyloxy)acetamide;
N-(1H-pyrazol-3-yl)-N-(thiophen-2-ylmethyl)-2-(p-tolyloxy)acetamide;
2-(4-fluorophenoxy)-N-(1H-pyrazol-3-yl)-N-(thiophen-2-ylmethyl)acetamide;
2-(2-hydroxy-4-methylphenoxy)-N-(1H-pyrazol-3-yl)-N-(thiophen-2-ylmethyl)-acetamide;
2-((2,3-dihydro-1H-inden-5-yl)oxy)-N-(1H-pyrazol-3-yl)-N-(thiophen-2-ylmethyl)-acetamide;
2-((2,3-dihydro-1H-inden-5-yl)oxy)-N-(1H-pyrazol-3-yl)-N-(thiazol-5-ylmethyl)-acetamide;
2-((5-methoxybenzofuran-2-yl)oxy)-N-(1H-pyrazol-3-yl)-N-(thiophen-2-ylmethyl)-acetamide; and
any combinations thereof.

12. The use of any one of claims 1 to 11, wherein the flavored product is a food product, a beverage product, an oral care product, or a pharmaceutical product.

13. The use of any one of claims 1 to 11, wherein the body care product is a cosmetic product, a skin care product, or a hair care product.

## Patentansprüche

1. Verwendung einer kühleverstärkenden Verbindung zum Verstärken einer kühlenden Wahrnehmung eines aromatisierten Produkts oder eines Körperpflegeprodukts, wobei die kühleverstärkende Verbindung ausgewählt ist aus der Gruppe bestehend aus: (E/Z)-2-Methyl-2-butenal, (E/Z)-2-Isopropyl-5-methyl-2-hexenal und beliebigen Kombinationen davon.

2. Verwendung gemäß Anspruch 1, wobei die kühleverstärkende Verbindung (E/Z)-2-Methyl-2-butenal ist.

3. Verwendung gemäß Anspruch 1, wobei die kühleverstärkende Verbindung (E/Z)-2-Isopropyl-5-methyl-2-hexenal ist.

4. Verwendung gemäß einem der Ansprüche 1 bis 3, wobei das aromatisierte Produkt oder das Körperpflegeprodukt einen kühlenden Stoff umfasst.

5. Verwendung gemäß Anspruch 4, wobei der kühlende Stoff (-)-Menthol, N-Ethyl-p-menthyl-3-carboxyamid, Cubebol, Isopulegol, 3-(L-Menthoxy)propan-1,2-diol, cis/trans-p-Menthan-3,8-diol, 3-(L-Menthoxy)-2-methylpropan-1,2-diol, 2-[2-(p-Menthan-3-yloxy)ethoxy]ethanol, Menthoxyethanol, (1R,2R,4R)-1-(2-Hydroxy-4-methylcyclohexyl)ethenon, (1R,2R,5R)-N-Ethyl-5-methyl-2-(prop-1-en-2-yl)cyclohexancarboxamid, N-(3-Hydroxy-4-methoxyphenyl)-2-isopropyl-5,5-dimethylcyclohexancarboxamid, N-[4-(Cyanomethyl)phenyl]-2-isopropyl-5,5-dimethylcyclohexancarboxamid, N-(2-Hydroxy-2-phenylethyl)-2-isopropyl-5,5-dimethylcyclohexan-1-carboxamid, L-Menthollactat, (-)-Menthylethylenglycolcarbonat, (-)-Menthon-1,2-glycerolketal, D/L-Menthon-1,2-glycerolketal, (-)-Menthyl-1-propylenglycolcarbonat, (-)-Menthyl-2-propylenglycolcarbonat, D/L-Menthyl-1-propylenglycolcarbonat, D/L-Menthyl-2-propylenglycolcarbonat, (1R,2S,5R)-N-(4-Methoxyphenyl)-5-methyl-2-(1-methylethyl)cyclohexancarboxamid, Menthylethylamidooxalat, (E)-3-Benzo[1,3]dioxol-5-yl-N,N-diphenyl-2-propenamid, 3,4-Methylendioxyzimtsäure, N,N-Diphenylamid, N,N-Dimethyl-(-)-menthylsuccinimid, (1R,2S,5R)-N-(4-(Carbamoylmethyl)phenyl)menthylcarboxamid, (-)-Menthylpyrrolidoncarboxylat, L-Phenylephrin-p-menthancarboxamid, (1R,2S,5R)-N-(4-(Cyanomethyl)phenyl)menthylcarboxamid, (1R,2S,5R)-N-(2-(Pyridin-2-yl)ethyl)menthylcarboxamid, 6-Isopropyl-3,9-dimethyl-1,4-dioxaspiro[4.5]decan-2-on, (1R,2R,4S)-Dihydroumbellulol, N-Ethyl-p-menthan-3-carboxamid, 2-Isopropyl-N,2,3-trimethylbutyramid, Ethyl-3-(p-menthan-3-carboxamido)acetat, N-Ethyl-2,2-diisopropylbutanamid, N-(1,1-Dimethyl-2-hydroxyethyl)-2,2-dimethylbutanamid, N-Cyclopropyl-5-methyl-2-isopropylcyclohexancarboncarboxamid, (-)-Menthylacetoacetat, (-)-Menthylsuccinat, (-)-Menthyl-(S)-3-hydroxybutyrat, (-)-Menthylglutarat, 1-[2-Hydroxyphenyl]-4-[2-nitrophenyl]-1,2,3,6-tetrahydropyrimidin-2-on, Di-(-)-menthylglutarat, N-(2-Hydroxyethyl)-2,3-dimethyl-2-isopropylbutanamid oder eine beliebige Kombination davon ist.

6. Verwendung gemäß Anspruch 5, wobei der kühlende Stoff ausgewählt ist aus der Gruppe bestehend aus: (-)-Menthol, N-Ethyl-p-menthyl-3-carboxyamid, Cubebol und beliebigen Kombinationen davon.

7. Verwendung gemäß einem der Ansprüche 1 bis 6, wobei die kühleverstärkende Verbindung in einem aromatisierten Produkt verwendet wird und das aromatisierte Produkt einen Süßstoff mit hoher Intensität umfasst.

8. Verwendung gemäß Anspruch 7, wobei der Süßstoff mit hoher Intensität Natriumsaccharin, Sucralose oder eine beliebige Kombination davon ist.

9. Verwendung gemäß einem der Ansprüche 1 bis 8, wobei das aromatisierte Produkt 4-Amino-5,6-dimethylthieno[2,3-d]pyrimidin-2(1H)-on oder ein Salz davon umfasst.

10. Verwendung gemäß einem der Ansprüche 1 bis 9, wobei das aromatisierte Produkt ferner einen Aroma, wie z.B. ein Minzearoma, umfasst.

11. Verwendung gemäß einem der Ansprüche 1 bis 10, wobei das aromatisierte Produkt oder das Körperpflegeprodukt ferner eine Verbindung umfasst, die ausgewählt ist aus der Gruppe bestehend aus:
N-Ethyl-N-(thiophen-2-ylmethyl)-2-(p-tolyloxy)acetamid;
N-(1H-Pyrazol-3-yl)-N-(thiophen-2-ylmethyl)-2-(p-tolyloxy)acetamid;
2-(4-Fluorphenoxy)-N-(1H-pyrazol-3-yl)-N-(thiophen-2-ylmethyl)acetamid;
2-(2-Hydroxy-4-methylphenoxy)-N-(1H-pyrazol-3-yl)-N-(thiophen-2-ylmethyl)acetamid;
2-((2,3-Dihydro-1H-inden-5-yl)oxy)-N-(1H-pyrazol-3-yl)-N-(thiophen-2-ylmethyl)acetamid;
2-((2,3-Dihydro-1H-inden-5-yl)oxy)-N-(1H-pyrazol-3-yl)-N-(thiazol-5-ylmethyl)acetamid;
2-((5-Methoxybenzofuran-2-yl)oxy)-N-(1H-pyrazol-3-yl)-N-(thiophen-2-ylmethyl)acetamid; und
beliebigen Kombinationen davon.

12. Verwendung gemäß einem der Ansprüche 1 bis 11, wobei das aromatisierte Produkt ein Lebensmittelprodukt, ein Getränkeprodukt, ein Mundpflegeprodukt oder ein pharmazeutisches Produkt ist.

13. Verwendung gemäß einem der Ansprüche 1 bis 11, wobei das Körperpflegeprodukt ein kosmetisches Produkt, ein Hautpflegeprodukt oder ein Haarpflegeprodukt ist.

## Revendications

1. Utilisation d'un composé améliorant la fraîcheur pour améliorer une sensation rafraîchissante d'un produit aromatisé ou d'un produit de soin corporel, le composé améliorant la fraîcheur étant choisi dans le groupe constitué par (E/Z)-2-méthyl-2-buténal, (E/Z)-2-isopropyl-5-méthyl-2-hexénal et une quelconque combinaison correspondante.

2. Utilisation selon la revendication 1, le composé améliorant la fraîcheur étant le (E/Z)-2-méthyl-2-buténal.

3. Utilisation selon la revendication 1, le composé améliorant la fraîcheur étant le (E/Z)-2-isopropyl-5-méthyl-2-hexénal.

4. Utilisation selon l'une quelconque des revendications 1 à 3, le produit aromatisé et/ou le produit de soin corporel comprenant une substance rafraîchissante.

5. Utilisation selon la revendication 4, la substance rafraîchissante étant (-)-menthol, N-éthyl-p-menthyl-3-carboxiamide, cubébol, isopulégol, 3-(L-menthoxy)propane-1,2-diol, cis/trans-p-menthane-3,8-diol, 3-(L-menthoxy)-2-méthylpropane-1,2-diol, 2-[2-(p-menthan-3-yloxy)éthoxy]éthanol, menthoxyéthanol, (1R,2R,4R)-1-(2-hydroxy-4-méthylcyclohexyl)éthénone, (1R,2R,5R)-N-éthyl-5-méthyl-2-(prop-1-en-2-yl)cyclohexanecarboxamide, N-(3-hydroxy-4-méthoxyphényl)-2-isopropyl-5,5-diméthylcyclohexanecarboxamide, N-[4-(cyanométhyl)phényl]-2-isopropyl-5,5-diméthylcyclohexanecarboxamide, N-(2-hydroxy-2-phényléthyl)-2-isopropyl-5,5-diméthyl-cyclohexane-1-carboxamide, lactate de L-menthol, carbonate de (-)-menthyléthylèneglycol, (-)-menthone 1,2-glycérol cétal, D/L-menthone 1,2-glycérol cétal, (-)-menthyl 1-propylène glycol carbonate, carbonate de (-)-menthyle et de 2-propylène glycol, carbonate de D/L-menthyle et de 1-propylène glycol, carbonate de D/L-menthyle et de 2-propylène glycol, (1R,2S,5R)-N-(4-méthoxyphényl)-5-méthyl-2-(1-méthyléthyl)cyclohexanecarboxamide, éthylamidooxalate de menthyle, (E)-3-benzo[1,3]dioxol-5-yl-N,N-diphényl-2-propénamide, acide 3,4-méthylènedioxycinnamique, N,N-diphénylamide, N,N-diméthyl(-)-menthylsuccinimide, (1R,2S,5R)-N-(4-(carbamoylméthyl)phényl)-menthylcarboxamide, pyrrolidonecarboxylate de (-)-menthyle, L-phényléphrine p-menthane carboxamide, (1R,2S,5R)-N-(4-(cyanométhyl)phényl)menthylcarboxamide, (1R,2S,5R)-N-(2-(pyridin-2-yl)éthyl)menthylcarboxamide, 6-isopropyl-3,9-diméthyl-1,4-dioxaspiro[4.5]décan-2-one, (1R,2R,4S)-dihydroumbellulol, N-éthyl-p-menthane-3-carboxamide, 2-isopropyl-N,2,3-triméthylbutyramide, 3-(p-menthane-3-carboxamido)acétate d'éthyle, N-éthyl-2,2-diisopropylbutanamide, N-(1,1-diméthyl-2-hydroxyéthyl)-2,2-diméthylbutanamide, N-cyclopropyl-5-méthyl-2-isopropyl-cyclohexanecarbonecarboxamide, (-)-acétoacétate de menthyle, (-)-succinate de menthyle, (-)-(S)-3-hydroxybutyrate de menthyle, glutarate de (-)-menthyle, 1-[2-hydroxyphényl]-4-[2-nitrophényl]-1,2,3,6-tétrahydropyrimidine-2-one, glutarate de di-(-)-menthyle, N-(2-hydroxyéthyl)-2,3-diméthyl-2-isopropylbutanamide, et de quelconques combinaisons correspondantes.

6. Utilisation selon la revendication 5, la substance rafraîchissante étant choisie dans le groupe constitué par : (-)-menthol, N-éthyl-p-menthyl-3-carboxiamide, cubébol, et de quelconques combinaisons correspondantes.

7. Utilisation selon l'une quelconque des revendications 1 à 6, le composé améliorant la fraîcheur étant utilisé dans un produit aromatisé, et le produit aromatisé comprenant un édulcorant à intensité élevée.

8. Utilisation selon la revendication 7, l'édulcorant à intensité élevée étant la saccharine sodique, le sucralose ou une quelconque combinaison correspondante.

9. Utilisation selon l'une quelconque des revendications 1 à 8, le produit aromatisé comprenant de la 4-amino-5,6-diméthyl-thiéno[2,3-d]pyrimidin-2(1H)-one, ou un sel correspondant.

10. Utilisation selon l'une quelconque des revendications 1 à 9, le produit aromatisé comprenant en outre un arôme, tel qu'un arôme de menthe.

11. Utilisation selon l'une quelconque des revendications 1 à 10, le produit aromatisé ou le produit de soin personnel comprenant en outre un composé choisi dans le groupe constitué par :
N-éthyl-N-(thiophén-2-ylméthyl)-2-(p-tolyloxy)acétamide ;
N-(1H-pyrazol-3-yl)-N-(thiophén-2-ylméthyl)-2-(p-tolyloxy)acétamide ;
2-(4-fluorophénoxy)-N-(1H-pyrazol-3-yl)-N-(thiophén-2-ylméthyl)acétamide ;
2-(2-hydroxy-4-méthylphénoxy)-N-(1H-pyrazol-3-yl)-N-(thiophén-2-ylméthyl)-acétamide ;
2-((2,3-dihydro-1H-indén-5-yl)oxy)-N-(1H-pyrazol-3-yl)-N-(thiophén-2-ylméthyl)acétamide ;
2-((2,3-dihydro-1H-indén-5-yl)oxy)-N-(1H-pyrazol-3-yl)-N-(thiazol-5-ylméthyl)acétamide ;
2-((5-méthoxybenzofuran-2-yl)oxy)-N-(1H-pyrazol-3-yl)-N-(thiophén-2-ylméthyl)acétamide ; et
de quelconques combinaisons correspondantes.

12. Utilisation selon l'une quelconque des revendications 1 à 11, le produit aromatisé étant un produit alimentaire, un produit de boisson, un produit de soins bucco-dentaires ou un produit pharmaceutique.

13. Utilisation selon l'une quelconque des revendications 1 à 11, le produit de soin personnel étant un produit cosmétique, un produit de soin de la peau ou un produit de soin capillaire.
